(19) Europäisches Patentamt
European Patent Office
Office européen des brevets

(11) Veröffentlichungsnummer: **0 180 136**
**B1**

(12) # EUROPÄISCHE PATENTSCHRIFT

(45) Veröffentlichungstag der Patentschrift: **02.01.91**

(21) Anmeldenummer: **85113442.9**

(22) Anmeldetag: **23.10.85**

(51) Int. Cl.[5]: **C 07 D 249/08,** C 07 D 233/60, C 07 D 233/61, C 07 D 405/12, C 07 D 295/10, C 07 C 49/84, C 07 C 49/80, C 07 C 49/76, C 07 D 303/08, C 07 C 321/22, C 07 C 321/28

(54) **Substituierte Azolylmethyl-cyclopropyl-carbinol-Derivate.**

(30) Priorität: **02.11.84 DE 3440116**
**22.06.85 DE 3522440**
**26.09.85 DE 3534310**

(43) Veröffentlichungstag der Anmeldung:
**07.05.86 Patentblatt 86/19**

(45) Bekanntmachung des Hinweises auf die Patenterteilung:
**02.01.91 Patentblatt 91/01**

(84) Benannte Vertragsstaaten:
**AT BE CH DE FR GB IT LI NL SE**

(56) Entgegenhaltungen:
**EP-A-0 054 974**
**EP-A-0 070 798**
**EP-A-0 086 173**
**EP-A-0 106 515**
**EP-A-0 111 234**
**FR-A-2 535 321**
**GB-A-2 136 423**

**JOURNAL OF ORGANIC CHEMISTRY, Band 48, 1983, Seiten 5133, 5134; K. OKUMA et al.: "Reaction of dimethyloxosulfonium methylide with epoxides preparation of oxetanes."**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Böckmann, Klaus, Dr.**
**Andreas Gryphius-Str. 7**
**D-5000 Köln 80 (DE)**
Erfinder: **Regel, Erik, Dl.**
**Untere Bergerheide 26**
**D-5600 Wuppertal 1 (DE)**
Erfinder: **Büchel, Karl Heinz, Prof.Dr.**
**Dabringhausener Str. 42**
**D-5093 Burscheid (DE)**
Erfinder: **Lürssen, Klaus, Dr.**
**August Kierspel-Str. 151**
**D-5060 Bergisch-Gladbach 2 (DE)**
Erfinder: **Konze, Jörg, Dr.**
**Magazinstr. 61**
**D-5000 Köln 90 (DE)**
Erfinder: **Brandes, Wilhelm, Dr.**
**Eichendorffstr. 3**
**D-5653 Leichlingen (DE)**

(56) Entgegenhaltungen:
**Rocz. Chem. Band 51, Nr. 1, S. 176-177**
**Tetrahedron, Band 37, Nr. 23, 1981, S. 3947**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 180 136 B1

**Beschreibung**

Die vorliegende Erfindung betrifft neue substituierte Azolylmethyl-cyclopropyl-carbinol-Derivate, mehrere Verfahren zu ihrer Herstellung sowie ihre Verwendung als Pflanzenwachstumsregulatoren und Fungizide.

Es ist bereits bekannt geworden, daß bestimmte Diazolyl-Derivate fungizide und pflanzenwachstumsregulierende Eigenschaften besitzen (vgl. EP—A—0 044 605). So lassen sich zum Beispiel 1,3-Di-(1,2,4-trizol-1-yl)-2-(2-chlorphenyl)-propan-2-ol, 1,3-Di-(1,2,4-triazol-1-yl)-2-(3-chlorphenyl)-propan-2-ol, 1,3-Di-(1,2,4-triazol-1-yl)-2-(4-chlorphenyl)-propan-2-ol und 1,3-Di(1,2,4-triazol-1-yl)-2-phenyl-propan-2-ol zur Regulierung des Pflanzenwachstums und zur Bekämpfung von Pilzen verwenden. Die Wirkung dieser Stoffe ist jedoch, insbesondere bei niedrigen Aufwandmengen und -konzentrationen, nicht immer ganz befriedigend.

Ferner sind aus der EP—A—0 086 173 schon fungizide und pflanzenwachstumsrgulierend wirksame Azolyl-Derivate bekannt, in denen ein substituierter Cyclopropyl-Rest als Substituent am zentralen Kohlenstoffatom enthalten sein kann. Es werden aber keine Verbindungen dieses Typs offenbart in denen das Carbinol-Kohlenstoffatom sowohl mit einem substituierten Cyclopropyl-Rest als auch mit einem gegebenenfalls substituierten Aryl-Rest verknüpft ist.

Schließlich werden in der EP—A—0 111 234 zahlreiche Azolyl-Verbindungen mit fungiziden Eigenschaften beschrieben. Jedoch werden keine Stoffe dieser Klasse erwähnt, die einen substituierten Cyclopropyl-Ring am zentralen Kohlenstoffatom aufweisen.

Es wurden nun neue substituierte Asolylmethyl-cyclopropylcarbinol-Derivate der Formel

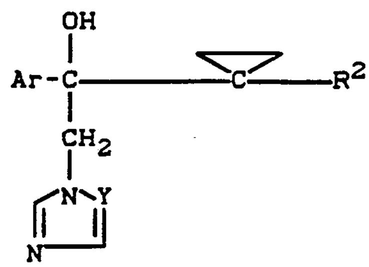

(I)

in welcher

Ar für Phenyl steht, das einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder

Ar für Naphthyl steht,

$R^2$ für Fluor, Chlor, Brom oder für die Gruppierungen —X—$R^3$ und —$NR^4R^5$ steht, worin

$R^3$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 18 Kohlenstoffatomen, gerdkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie für Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl oder Phenoxy, oder

$R^3$ für den Rest der Formel

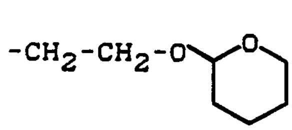 steht,

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann, und

X für Sauerstoff, Schwefel, eine SO- oder eine $SO_2$-Gruppe steht, und

Y für Stickstoff oder eine CH-Gruppe steht,

sowie deren Säureadditions-Salze und Metallsalz-Komplexe gefunden.

Weiterhin wurde gefunden, daß man substituierte Azolylmethyl-cyclopropyl-carbinol-Derivate der Formel (I) sowie deren Säureadditions-Salze und Metallsalz-Komplexe erhält, wenn man

a) in einer *ersten Stufe* Aryl-cyclopropyl-ketone der Formel

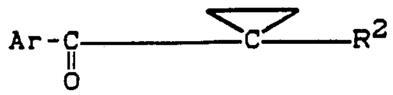

(II)

in welcher Ar und $R^2$ die oben angegebene Bedeutung haben,
mit Dimethyloxosulfonium-methylid der Formel

$$(CH_3)_2\ {}^{\delta+}SO^{\delta-}CH_2 \qquad (III)$$

in Gegenwart eines Verdünnungsmittels umsetzt und die dabei entstehenden Aryl-cyclopropyloxirane der Formel

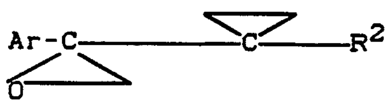

(IV)

in welcher Ar und $R^2$ die oben angegebene Bedeutung haben,
in einer *zweiten Stufe* mit Azolen der Formel

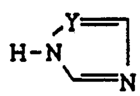

(V)

in welcher Y die oben angegebene Bedeutung hat,
in Gegenwart eines Verdünnungsmittels und in Gegenwart einer Base umsetzt, oder

b) Azolyl-keto Verbindungen der Formel

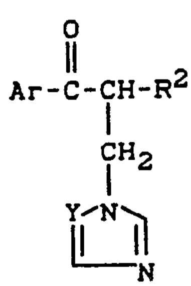

(VI)

in welcher $R^2$, Ar und Y die oben angegebene Bedeutung haben
mit Dimethyloxosulfonium-methylid der Formel

$$(CH_3)_2\ {}^{\delta+}SO^{\delta-}CH_2 \qquad (III)$$

in Gegenwart eines Verdünnungsmittels umsetzt oder

c) Azolylmethyl-thio-cyclopropyl-carbinol-Derivate der Formel

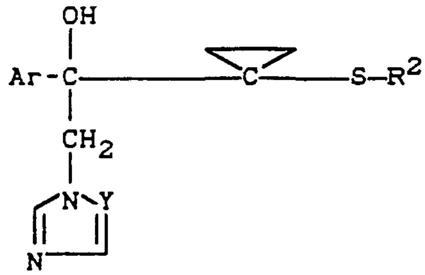

(Ia)

in welcher Ar, $R^3$ und Y die oben angegebene Bedeutung haben,
mit Oxidationsmitteln gegebenenfalls in Gegenwart eines Verdünnungsmittels umsetzt,
und gegebenenfalls an die so erhaltenen Verbindungen der Formel (I) eine Säure oder ein Metallsalz addiert.

Schließlich wurde gefunden, daß die neuen substituierten Azolylmethyl-cyclopropyl-carbinol-Derivate der Formel (I) sowie deren Säureadditions-Salze und Metallsalz-Komplexe starke pflanzenwuchsregulierende und fungizide Eigenschaften besitzen.

Überraschenderweise zeigen die erfindungsgemäßen Stoffe eine bessere pflanzenwuchsregulierende und fungizide Wirksamkeit als die konstitutionell ähnlichen, aus dem Stand der Technik vorbekannten Diazolyl-Derivate 1,3-Di-(1,2,4-triazol-1-yl)-2-(2-chlorphenyl)-propan-2-ol, 1,3-Di(1,2,4-triazol-1-yl)-2-(3-chlorphenyl)-propan-2-ol, 1,3-Di-(1,2,4-triazol-1-yl)-2-(4-chlorphenyl)-propan-2-ol und 1,3-Di-(1,2,4-triazol-1-yl)-2-phenyl-propan-2-ol, die für die gleichen Indikationen verwendbar sind.

Die erfindungsgemäßen substituierten Azolylmethyl-cyclopropan-carbonol-Derivate sind durch die Formel (I) allgemein definiert.

Bevorzugt sind diejenigen Verbindungen der Formel (I), in denen

Ar für gegebenenfalls einfach bis dreifach, insbesondere einfach oder zweifach, gleich oder verschieden substituiertes Phenyl steht, wobei als Substituenten genannt seien: Fluor, Chlor, Methyl, Isopropyl, tert.-Butyl, Methoxy, Methylthio, Trifluormethyl, Trifluormethoxy, Trifluormethylthio, sowie Phenyl und Phenoxy, oder

Ar für Naphthyl steht,

$R^2$ für Fluor, Chlor, Brom oder für die Gruppierungen —X—$R^3$ und —$NR^4R^5$ steht, worin

$R^3$ für geradkettiges oder verzweigtes Alkyl mit 1 bis 12 Kohlenstoffatomen, Cycloalkyl mit 5 bis 7 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, Hydroxyalkyl mit 1 bis 12 Kohlenstoffatomen, Alkylthioalkyl mit 1 bis 4 Kohlenstoffatomen in der Alkylthiogruppe und 1 bis 4 Kohlenstoffatomen in der Alkylgruppe, Carboxyalkyl mit 1 bis 12 Kohlenstoffatomen im Alkylteil, Alkoxycarbonylalkyl mit 1 bis 4 Kohlenstoffatomen im Alkoxyteil und 1 bis 4 Kohlenstoffatomen im Alkylteil, sowie für jeweils gegebenenfalls einfach bis dreifach, insbesondere einfach oder zweifach, gleichartig oder verschieden substituiertes Phenyl oder Benzyl steht, wobei als Substituenten die oben bei Ar bereits genannten bevorzugten Phenylsubstituenten in Frage komen, oder

$R^3$ für den Rest der Formel

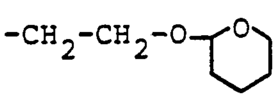

steht,

$R^4$ und $R^5$ unabhängig voneinander für Wasserstoff, Methyl, Ethyl, n-Propyl, Isopropyl, n-Butyl oder Isobutyl stehen oder

$R^4$ und $R^5$ gemeinsam mit dem Stickstoffatom, an das sie gebunden sind, für jeweils gegebenenfalls durch Methyl oder Ethyl substituiertes Piperidinyl, Piperazinyl oder Morpholinyl stehen, und

X für Sauerstoff, Schwefel, eine SO— oder eine $SO_2$-Gruppe steht, und

Y für Stickstoff oder eine CH-Gruppe steht.

Bevorzugte erfindungsgemäße Verbindungen sind auch Additionsprodukte aus Säuren und denjenigen substituierten Azolylmethyl-cyclopropyl-carbinol-Derivaten der Formel (I), in dene Ar, $R^2$ und Y die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure,

Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salze von Metallen der II. bis IV. Haupt- und der I. und II. sowie IV. bis VIII. Nebengruppe des Periodensystems und denjenigen substituierten Azolylmethyl-cycloproyl-carbinol-Derivaten der Formel (I), in denen Ar, $R^2$ und Y diejenigen Bedeutungen haben, die bereits vorzugsweise für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 1-(4-Chlorbenzoyl)-1-ethylthio-cyclopropan und Dimethyloxosulfonium-methylid als Ausgangsstoffe und 1,2,4-Triazol als Reaktonskomponente, so kann der Verlauf des erfindungsgemäßen Verfahren (a) durch das folgende Formelschema wiedergegeben werden:

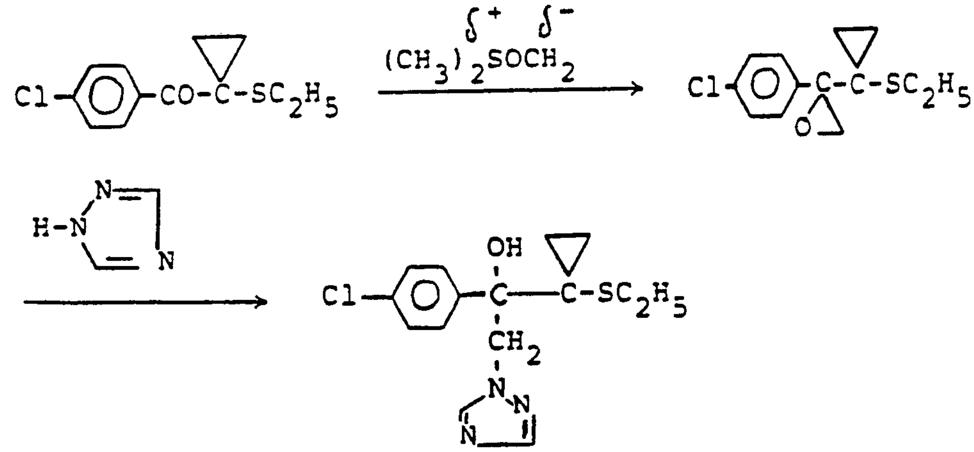


Verwendet man beispielsweise 1-(4-Chlorphenoxy)-2-(1,2,4-triazol-1-yl)-propiophenon und Dimethyloxosulfonium-methylid als Ausgangsstoffe, so kann der Verlauf des erfindungsgemäßen Verfahrens (b) durch das folgende Formelschema wiedergegeben werden:

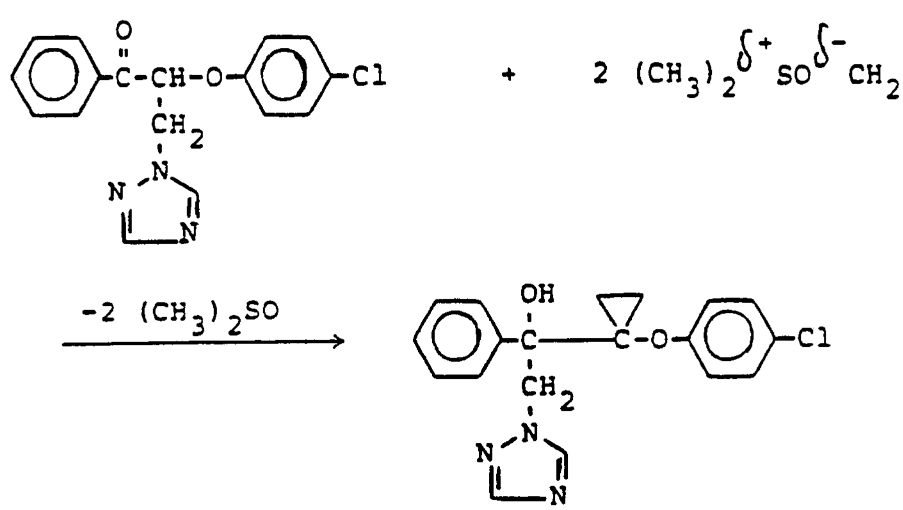


Verwendet man beispielsweise 1-(4-Chlorphenyl)-1-[1-(ethylthio)-1-cyclopropyl]-2-(1,2,4-triazol-1-yl)-1-ethanol und Wasserstoffperoxid in Eisessig als Ausgangsstoffe, so kann der Verlauf des erfindungsgemäßen Verfahrens (c) durch das folgende Formelschema wiedergegeben werden:

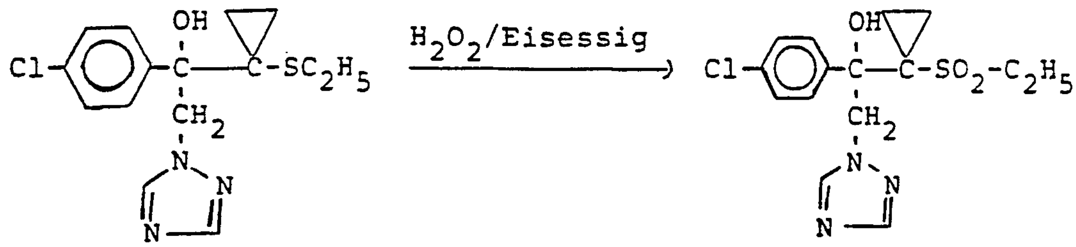


Die für das erfindungsgemäße Verfahren (a) als Ausgangsstoffe zu verwendenden Aryl-cyclopropyl-Ketone sind durch die Formel (II) allgemein definiert. In dieser Formel haben Ar und $R^2$ vorzugsweise die Bedeutungen, die bereits im Zusammenhang mit der Beschreibung der erfindungsgemäßen Stoffe der Formel (I) vorzugsweise für diese Substituenten genannt wurden.

Die Aryl-cyclopropyl-Ketone de Formel (II) sind zum Teil bekannt (vgl. Rocz. Chem. *51*, 176—177 (1977) und Tetrahedron *37*, 3947 (1981)).

Sie lassen sich herstellen, indem man Arylhalogenpropyl-ketone der Formel

Ar—CO—CH—$CH_2CH_2$—Hal'' (VIII)
|
Hal'

in welcher

Ar die oben angegebene Bedeutung hat,
Hal' für Fluor, Chlor ode Brom steht und
Hal'' für Brom oder Chlor steht,
α) mit Verbindungen der Formel

$HR^6$ (IX)

in welcher

$R^6$ für die Gruppierungen —$XR^3$ und —$NR^4R^5$ steht, worin $R^3$, $R^4$, $R^5$ und X die oben angegebenen Bedeutungen haben,

in Gegenwart eines Verdünnungsmittels und in Gegenwart eine Base umsetzt, oder

β) direkt in Gegenwart eines Verdünnungsmittels und in Gegenwart einer Base erhitzt.

Die Aryl-halogenpropyl-ketone der Formel (VIII) sind bekannt oder lassen sich nach im Prinzip bekannten Verfahren in einfacher Weise herstellen (vgl. DE—A 25 21 104, DE—A 23 20 355 und DE—A 23 51 948). So können zum Beispiel die Aryl-halogenpropylketone der Formel (VIII), in denen Hal' für Fluor steht, hergestellt werden, indem man die entsprechenden Verbindungen der Formel (VIII), in denen Hal' für Brom steh, mit Alkalimetallfluoridine, wie Natrium- oder Kaliumflorid, in Gegenwart eines inerten organischen Verdünnungsmittels, wie zum Beispiel Benzol, und in Gegenwart eines makrocyclischen Ethers, wie zum Beispiel 18-Krone-6, umsetzt (vgl. Herstellungsbeispiele).

Die Verbindungen der Formel (IX) sind bekannt oder lassen sich nach im Prinzip bekannten Methoden herstellen.

Verfährt man bei dem obigen Verfahren zur Herstellung von Aryl-cyclopropyl-ketonen der Formel (II) nach der Methode (β), so erhält man diejenigen Verbindungen der Formel (II), in denen $R^2$ für Halogen steht.

Bei dem obigen Verfahren zur Herstellung der Arylcyclopropyl-ketone der Formel (II) kommen als Verdünnungsmittel sowohl beim Arbeiten nach der Variante (α) als auch nach der Variante (β) alle unter den Reaktionsbedingungen inerten organischen Solventien in Frage. Vorzugsweise verwendbar sind Alkohole, wie Methanol, Ethanol, Methoxyethanol, Propanol oder tert.-Butanol, ferner Ketone, wie Aceton und 2-Butanon, außerdem Nitrile, wie Acetonitril, weiterhin Ester, wie Essigester, darüber hinaus Ether, wie Dioxan, aromatische Kohlenwasserstoffe, wie Benzol oder Toluol, und auch Amide, wie Dimethylformamid.

Als Basen kommen bei der Herstellung von Arylcyclopropyl-ketonen der Formel (II) sowohl beim Arbeiten nach der Variante (α) als auch nach der Variante (β) alle üblicherweise verwendbaren anorganischen und organischen Basen in Betracht. Hierzu gehören vorzugsweise Alkalicarbonate, wie Natrium- und Kaliumcarbonat, Alkalihydroxide, wie Natrium- und Kaliumhydroxid, Alkalialkoholate, wie Natrium- und Kalium-methylat, -ethylat und -tert.-butylat; Alkalihydride, wie Natriumhydrid, sowie niedere tertiäre Alkylamine, Cycloalkylamine und Aralkylmine, wie insbesondere Triethylamin.

Die Reaktionstemperaturen können bei der Durchführung des obigen Verfahrens zur herstellung der Arylcyclopropylketone der Formel (II) sowohl in der Variante (α) als auch in der Variante (β) in einem größeren Bereich variiert werden. Im allgemeinen arbeitet man bei Temperaturen zwischen 0° und 200°C, vorzugsweise zwischen 20°C und 150°C.

Bei der Herstellung von Aryl-cyclopropyl-ketonen der Formel (II) nach der obigen Verfahrensvariante (α) setzt man vorzugsweise auf 1 Mol Aryl-halogenpropyl-keton der Formel (VIII) 1 bis 2 Mol an Verbindung der Formel (IX) und 1 bis 2 Mol an Base ein. Die Isolierung der Verbindungen der Formel (II) erfolgt in üblicher Weise.

Die Aryl-cyclopropyl-ketone der Formel (II) sind nicht nur als Ausgangsstoffe zur Herstellung der erfindungsgemäßen Verbindungen der Formel (I) von Interesse, sondern stellen darüber hinaus auch wertvolle Zwischenprodukte zur Synthese anderer Substanzen dar.

Das bei den erfindungsgemäßen Verfahren (a) und (b) jeweils als Reaktionskomponente benötigte Dimethyloxosulfonium-methylid der Formel (III) ist bekannt (vgl. J. Am. Chem. Soc. *87*, 1363—1364 (1965)). Es wird bei der obigen Umsetzung in frisch zubereitetem Zustand verarbeitet, indem man es in situ durch Umsetzung von Trimethyloxosulfoniumjodid mit Natriumhydrid oder Natriumamid, insbesondere mit Kalium-tert.-butylat oder Natriummethylat, in Gegenwart eines Verdünnungsmittels erzeugt.

Die außerdem für die zweite Stufe des erfindungsgemäßen Verfahrens (a) als Ausgangsstoffe benötigten Azole der Formel (V) sind allgemein bekannte Verbindungen der organischen Chemie.

Die bei dem erfindungsgemäßen Verfahren (a) als Zwischenprodukte auftretenden Aryl-cyclopropyl-oxirane der Formel (IV) sind noch nicht bekannt. Sie stellen allgemein interessante Zwischenprodukte dar.

Die für das erfindungsgemäße Verfahren (b) als Ausgangsstoffe zu verwendenden Azolyl-keto-Verbindungen sind durch die Formel (VI) allgemein definiert. In dieser Formel (VI) stehen $R^2$, Ar und Y vorzugsweise für diejenigen Reste die bereits im Zusammenhang mit der Beschreibung der erfindungsgemäßen Stoffe der Formel (I) als bevorzugt für diese Substituenten genannt wurden.

Die Azolyl-keto-Verbindungen der Formel (VI) sind bisher nur teilweise bekannt (vgl. DE—A 23 48 663).

Die Azolyl-keto-Verbindungen der Formel

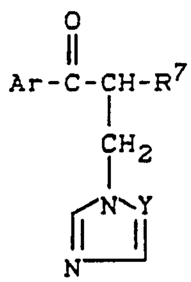

(VIa)

in welcher

Ar für Phenyl steht, das einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

Ar für Naphthyl steht,

Y für Stickstoff oder eine CH—Gruppe steht, und

$R^7$ für Fluor, Chlor, Brom oder für die Gruppierungen $—XR^8$ und $—NR^4R^5$ steht, worin

X für Sauerstoff, Schwefel, eine SO— oder eine $SO_2$-Gruppe steht,

$R^8$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 18 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie for Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

$R^8$ für den Rest der Formel

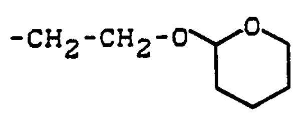 steht, und

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann;

sind neu.

Die Azolyl-keto-Verbindungen der Formel (VIa) lassen sich herstellen, indem man Ketone der Formel

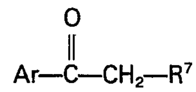

(X)

in welcher Ar und $R^7$ die oben angegebene Bedeutung haben,
mit Hydroxymethylazolen der Formel

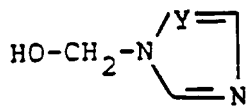

(XI)

in welcher Y die oben angegebene Bedeutung hat,
in Gegenwart eine Verdünnungsmittels und in Gegenwart eines Katalysators umsetzt.

Die bei der Herstellung der Arylketo-Verbindungen der Formel (VIa) als Ausgangsstoffe benötigten Ketone der Formel (X) sind bekannt oder lassen sich nach im Prinzip bekannen Methoden herstellen.

Die außerdem bei der Herstellung der Aryl-keto-Verbindungen de Formel (VIa) nach dem obigen Verfahren als Ausgangsstoffe benötigten Hydroxymethylazole der Formel (XI) sind bekannt (vgl. EP—A 0 006 102 und Chem. Heterocycl. Comp. *1980*, 189).

Als Verdünnungsmittel kommen für das obige Verfahren zur Herstellung der Azolyl-keto-Derivaten der Formel (VIa) vorzugsweise inerte organische Lösungsmittel in Frage. Hierzu gehören vorzugsweise Alkohole, wie Methanol und Phenol, ferner Ether wie Tetrahydrofuran und Dioxan, weiterhin aromatische Kohlenwasserstoffe, wie Benzol und Toluol, und außerdem halogenierte aliphatische und aromatische Kohlenwasserstoffe, wie Methylenchlorid, Chloroform, Chlorbenzol und Dichlorbenzol.

Das Verfahren zur Herstellung der Azolyl-keto-Derivate der Formel (VIa) wird in Gegenwart eines Katalysators durchgeführt. Man kann dabei alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen wie Pyridin und Piperidin, sowie insbesondere Piperidinacetat.

Die Reaktionstemperaturen können bei der Durchführung dieses Verfahrens in einem größeren Bereich variiert werden. Im allgemeinen arbeitet man zwischen 20 und 160°C, vorzugsweise bei der Siedetemperatur des jeweiligen Lösungsmittels.

Bei der Durchführung dieses Verfahrens setzt man auf 1 Mol Keton der Formel (X) 1 bis 1,5 Mol Hydroxymethylazol der Formel (XI) und katalytische bis 0,2 molare Mengen an Katalysator ein.

Die Azolyl-keto-Derivate der Formel (VI) stellen interessante Zwischenprodukte dar und zeigen in entsprechenden Aufwandmengen bzw. -konzentrationen auch fungizide und pflanzenwachstumsregulierende Eigenschaften.

Die für das erfindungsgemäße Verfahren (c) als Ausgangsstoffe zu verwendenden Azolylmethyl-thiocyclopropylcarbinol-Derivate der Formel (Ia) sind erfindungsgemäße Verbindungen.

Als Reaktionskomponenten kommen bei dem erfindungsgemäßen Verfahren (c) alle für derartige Umsetzungen überlichen Oxidationsmittel in Betracht. Vorzugsweise verwenbar sind Wasserstoffperoxid und Persäuren, wie m-Chlorperbenzoesäure und Peressigsäure.

Als Verdünnungsmittel kommen für die erste Stufe des erfindungsgemäßen Verfahrens (a) inerte organische Lösungsmittel in Frage. Hierzu gehören vorzugsweise Ether, wie Tetrahydrofuran oder Dioxan, aliphatische und aromatische Kohlenwasserstoffe, wie insbesondere Benzol, Toluol oder Xylol; sowie Dimethylsulfoxid.

Die Reaktionstemperaturen können bei der Durchführung de ersten Stufe des erfindungsgemäßen Verfahrens (a) in einem größeren Bereich variiert werden. Im allgemeinen arbeitet man zwischen 0 und 100°C, vorzugsweise zwischen 10 und 60°C.

Bei der Durchführung der ersten Stufe des erfindungsgemäßen Verfahrens (a) setzt man auf 1 Mol Aryl-cyclopropyl-keton der Formel (II) vorzugsweise 1 bis 3 Mol Dimethyloxosulfonium-methylid der Formel (III) ein, in-situ erzeugt aus Trimethyloxosulfoniumjodid in Dimethylsulfoxid und Kalium-tert.-butylat. Die Isolierung der Zwischenprodukte der Formel (IV) erfolgt in allgemein übliche Art und Weise.

Als Verdünnungsmittel kommen für die zweite Stufe des erfindungsgemäßen Verfahrens (a) inerte organische Lösungsmittel in Frage. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid; sowie Hexamethylphosphorsäuretriamid.

Die zweite Stufe des erfindungsgemäßen Verfahrens (a) wird in Gegenwart einer Base durchgeführt. Dabei kommen alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei de Durchführung de zweiten Stufe des erfindungsgemäßen Verfahrens (a) in einem größeren Bereich variiert werden. Im allgemeinen arbeitet man bei Temperaturen zwischen 0°C und 200°C, vorzugsweise zwischen 60°C und 150°C.

Bei der Durchführung der zweiten Stufe des erfindungsgemäßen Verfahrens (a) setzt man vorzugsweise auf 1 Mol Oxiran der Formel (IV) 1 bis 2 Mol Azol und 1 bis 2 Mol Base ein. Die Isolierung der Endprodukte erfolgt in allgemein üblicher Weise.

Die Reaktionsbedingungen bei der Durchführung des erfindungsgemäßen Verfahrens (b) entsprechen denen für die Durchführung der ersten Stufe des Verfahrens (a).

Bei der Durchführung des erfindungsgemäßen Verfahrens (c) setzt man auf 1 Mol der erfindungsgemäßen Verbindungen der Formel (Ia) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Essigsäure oder Acetanhydrid bei Temperaturen zwischen −30°C bis +30°C, entstehen vorzugsweise die erfindungsgemäßen Verbindungen der Formel (I) mit de —SO-Gruppierung. Bei Überschuß an Oxidationsmittel und höheren Temperaturen (10 bis 80°C) entstehen vorzugsweise die erfindungsgemäßen Verbindungen der Formel (I) mit der —$SO_2$-Gruppierung. Die Isolierung der Oxidationsprodukte erfolgt in üblicher Weise.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen de Säure, z.B. Chlorwassersoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffee hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwachsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachhstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlabut es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie. z.B. Zucker in Zuckerrüben oder Zuckerrohr, mit Wachstumsregulatoren vor oder nach der Ernte zu hemmen. Außerdem läßt sich die Produktion oder der Abfluß von sekundären

Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung de Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfal steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich is es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas ode Zierpflanzen in Gärtnereien, zu einem Zeitpunkt keimen, austreiben oder blühen, an dem sie normalerweie hierzu keine Bereitschaft zeigen. Eine Verzögerung des Austriebes von Knospen oder der Keimun von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstofe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Getreide- und Reiskrankheiten sowie von Venturia-Arten, wie Venturia inaequalis, eingesetzt werden. Außerdem zeigen die erfindungsgemäßen Stoffe ein breites und gutes fungizides invitro-Wirkungsspektrum.

Die Wirkstofe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hegestellt, z.B. duch Vermischen der Wirkstoffe mit Streckmitteln, also flüssigen Lösungsmitteln, unter Druck stehenden verflüssigten Gasen und/oder festen Trägerstoffen, gegebenenfalls unter Vewendung von obeflächenaktiven Mitteln, also Emulgiermitteln und/ oder Dispegiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder

Trägerstoffen sind solche Flüssigkeiten gemeint, welche bei normaler Temperatur und unter Normaldruck gasförmig sind, z.B. Aerosol-Treibgase, wie Halogenkohlenwassersoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionnierte natürliche Gesteine wie Calcit, Marmor, Bims, Sepiolith, Dolomit sowie synthetische Granulate aus anorganischen und organischen Mehlen sowie Granulate aus oranischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen ode den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,0001 bis 0,1 Gew.-%, vorzugsweise von 0,0001 bis 0,02%, am Wirkungsort erforderlich.

Die Herstellung und die Verwendung der erfindungsgemäßen Stoffe geht aus den folgenden Beispielen hervor.

Herstellungsbeispiele

Beispiel 1

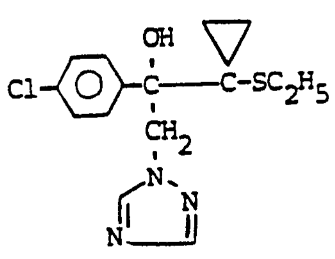

(I-1)

(Verfahren a)

1. Stufe

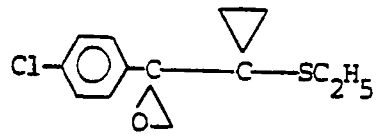

(IV-1)

Zu einer Mischung aus 6,4 g Natriumhydrid (80 %ig) und 44,2 g Trimethylsulfoxoniumjodid tropft man bei 10°C 170 ml trockenes Dimethylsulfoxid zu und läßt eine Stunde bei Raumtemperatur nachrühren. Anschließend versetzt man tropfenweise mit 40 g (0,167 Mol) 1-(4-Chlorbenzoyl)-1-ethylthio-cyclopropan in 50 ml Dimethylsulfoxid. Das Reaktoinsgemisch wird zwei Tage bei Raumtemperatur gerührt. Danach gießt man auf 500 g Eis, extrahiert mehrmals mit Essigester, wäscht die vereinigten organischen Phasen mit Wasser, trocknet über Natriumsulfat und engt ein. Man erhält 40,5 g (95,5% der Theorie) 1-[1-(4-Chlorphenyl)-oxiranyl]-1-ethylthio-cyclopropan als Öl, das direkt weitere umgesetzt wird.

2. Stufe

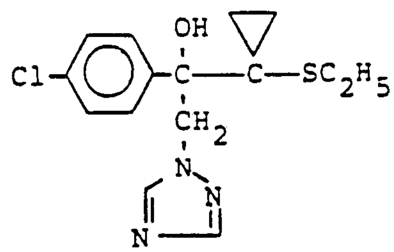

(I-1)

Zu einer siedenden Mischung aus 33,5 g 1,2,4-Triazol und 22g Kaliumcarbonat in 150 ml Acetonitril tropft man eine Lösung von 40,5 g (0,16 Mol) 1-1-(4-Chlorphenyl)-oxiranyl]-1-ethylthio-cyclopropan (vgl. Bsp.IV—1) in 50 ml Acetonitril und erhitzt das Reaktionsgemisch acht Stunden unter Rückfluß. Man läßt abkühlen, versetzt mit 400 ml Wasser und saugt das kristalline Produkt ab. Nach dem Umkristallisieren aus Ethanol/Wasser erhält man 26,5 g (51% der Theorie) 1-(4-Chlorphenyl)-1-[1-(ethylthio)-1-cyclopropyl]-2-(1,2,4-triazol-1-yl)-1-ethanol vom Schmelzpunkt 165°C.

Herstellung des Ausgangsproduktes

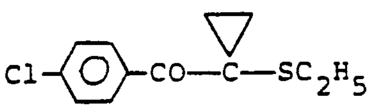

(II-1)

Zu einer Lösung von 100 g (0,338 Mol) 4-Chlorphenyl-(1-brom-3-chlorpropyl)-keton und 21 g Ethylthiol in 200 ml Dimethylformamid tropft man bei 0°C bis 10°C eine Lösung von 38 kg Kaliumhydroxid in 250 ml Methanol. Man läßt eine Stunde bei Raumtemperatur und eine Stunde unter Rückfluß nachrühren. Anschließend wird im Vakuum eingeengt und der Rückstand in einem Gemisch von Wasser und Methylenchlorid aufgenommen. Die oranische Phase wird abgetrennt, mit Wasser gewaschen, über Natriumsulfat getrocknet und im Vakuum eingeengt. Der Rückstand wird im Hochvakuum destilliert. Man erhält 70,1 g (86% d.Th.) 1-(4-Chlorbenzoyl)-1-ethylthio-cyclopropanon vom Siedepunkt $Kp_{0,15}$ = 127°C.

Beispiel 2

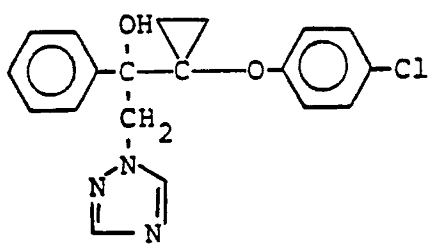

(I-2)

(Verfahren b)

19,7 g (0,06 Mol) 1-(4-Chlorphenoxy)-2-(1,2,4-triazol-1-yl)-propiophenon werden portionsweise

eingetragen in eine Lösung von Dimethylsulfoxoniummethylid in 70 ml Dimethylsulfoxid [hergestellt aus 27,6 g (0,125 Mol) Trimethylsulfoxoniumjodid und 14,03 g (0,125 Mol) Kalium-tert.-butylat].

Das Gemisch wird 6 Stunden bei 60°C gerührt, danach mit 1000 ml Wasser verdünnt, das abgeschiedene Öl in Chloroform aufgenommen, über Natriumsulfat getrocknet, eingeengt und chromatographisch (Kieselgel F 60 Merck/Laufmittel: Chloroform) gereinigt. Das verbleibende Öl kristallisiert beim Verrühren mit Acetonitril. Man erhält 1,7 g (8% der Theorie) an 1-(4-Chlorphenoxy)-1-[1-hydroxy-1-phenyl-2-(1,2,4-triazol-1-yl)]-cyclopropan vom Schmelzpunkt 170°C.

Herstellung des Vorproduktes

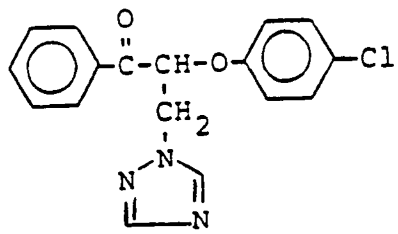

(VI-1)

29,4 g (0,12 Mol) ω-(4-Chlorphenoxy)-acetophenon und 11,9 g (0,12 Mol) 1,2,4-Triazol-1-yl-methylalkohol in 200 ml Toluol werden nacheinander mit 10,8 g (0,18 Mol) Essigsäure und 1,8 ml (0,018 Mol) Piperidin versetzt und am Wasserabscheider gekocht, bis die abgeschiedene Wassermenge konstant bleibt. Die Toluollösung wird anschließend mit Wasser gewaschen, über Natriumsulfat getrocknet, eingeengt und der ölige Rückstand wird chromatographisch gereinigt (Kieselgel F 60 Merck/Laufmittel Chloroform). Man erhält 20,8 g (53% der Theorie) an 1-(Chlorophenoxy)2-(1,2,4-triazol-1-yl)-propiophenon vom Schmelzpunkt 126°C.

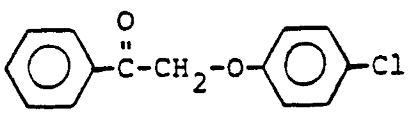

(X-1)

Eine Lösung von 154,6 g (1 Mol) ω-Chloracetophenon in 250 ml Butanon wird in eine siedende Lösung von 129 g (1 Mol) 4-Chlorphenol, 140 g (1 Mol) Kaliumcarbonat und 2 g Kaliumjodid in 800 ml Butanon getropft, und das Gemisch wird nach beendeter Zugabe weitere 12 Stunden unter Rückfluß gekocht. Die erhaltene Reaktionsmischung wird filtriert und eingedampft. Man erhält 179 g (73% der Theorie) an ω-(4-Chlorphenoxy)-acetophenon vom Schmelzpunkt 97°C.

## Beispiel 3

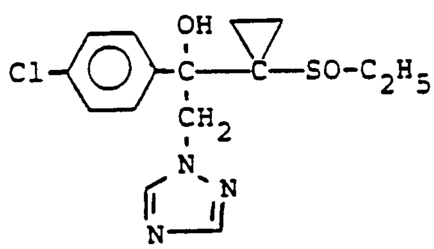

(I-3)

(Verfahren c)

5 g (0,0155 Mol) 1-(4-Chlorphenyl)-1-[1-(ethylthio)-1-cyclopropyl]-2-(1,2,4-triazol-1-yl)-1-ethanol (vgl. Beispiel 1) werden mit 3,3 g m-Chlorperbenzoesäure (80—90 %ig) in 40 ml Methylenchlorid bei Raumtemperatur über Nacht und anschließend eine Stunde unter Rückfluß gerührt. Anschließend wird zweimal mit je 20 ml 5 %iger wäßriger Natronlauge und zweimal mit Wasser gewaschen. Die organische Phase wird über Natriumsulfat getrocknet und im Vakuum eingeengt. Der Rückstand wird aus Glykolmonomethylether-acetat umkristallisiert. Man erhält 3 g (57% der Theorie) 1-(4-Chlorphenyl)-1-[1-(ethylsulfinyl)-1-cyclopropyl]-2-(1,2,4-triazol-1-yl)-1-ethanol vom Siedepunkt 185°C.

Beispiel 4

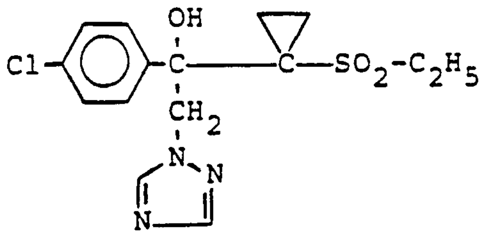

(I-4)

(Verfahren c)

3,2 g (0,01 Mol) 1-(4-Chlorphenyl)-1-[1-(ethylthio)-1-cyclopropyl]-2-(1,2,4-triazol-1-yl)-1-ethanol (vgl. Beispiel 1) werden in 20 ml Eisessig gelöst und mit 4 ml 30 %igem Wasserstoffperoxid sechs Stunden bei 70°C gerührt. Anschließend versetzt man mit 100 ml Eiswasser, neutralisiert mit konzentrierte wäßrier Natronlauge und saugt den kristallinen Feststoff ab. Man erhält 3,4 g (96% der Theorie) 1-(4-Chlorphenyl)-[1-(ethylsulfonyl)-1-cyclopropyl]-2-(1,2,4-triazol-1-yl)-1-ethanol vom Schmelzpunkt 215°C.

Nach den in den vorstehenden Beispielen beschriebenen Methoden und den Angaben zu den erfindungsgemäßen Verfahren werden auch die in der folgenden Tabelle 1 aufgeführten Stoffe der Formel (I) hergestelt.

Tabelle 1

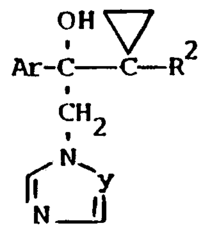

(I)

| Verb. Nr. | Ar | Y | $R^2$ | Schmelzpunkt (°C) |
|---|---|---|---|---|
| I-5 | 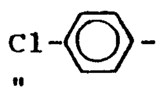 | N | 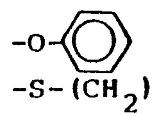 | 139 |
| I-6 | " | " | $-S-(CH_2)_3CH_3$ | 129 |
| I-7 | " | " | $-SO_2-(CH_2)_3CH_3$ | 136 |
| I-8 | " | " | F | 128 |
| I-9 | " | " | 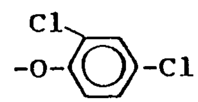 | 167 |
| I-10 | " | CH | 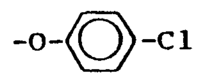 | 211 |
| I-11 | " | N | 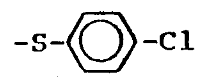 | 137 |
| I-12 | 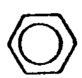 | " | 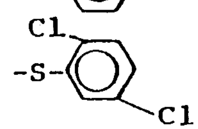 | 161 |
| I-13 | 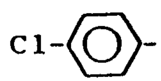 | " | 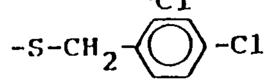 | 183 |

Tabelle 1 (Fortsetzung)

| Verb. Nr. | Ar | Y | $R^2$ | Schmelzpunkt |
|---|---|---|---|---|
| I-14 | 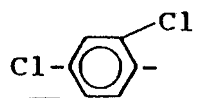 | N | 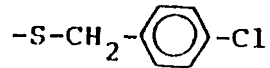 | |
| I-15 | 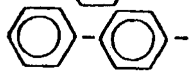 | " | 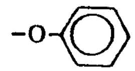 | 121 |
| I-16 | 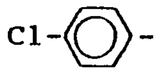 | " | $-SCH_3$ | 124 |
| I-17 | " | CH | $-SCH_3$ | 159 |
| I-18 | " | N | $-SO_2-CH_3$ | 193 |
| I-19 | " | " | $-SO_2-C_2H_5$ | 185 |
| I-20 | " | CH | $-S-C_2H_5$ | 158 |
| I-21 | " | N | $-S(CH_2)_2-CH_3$ | 131 |
| I-22 | " | CH | $-S-(CH_2)_2-CH_3$ | 133 |
| I-23 | " | N | $-S-CH_2-CH=CH_2$ | 134 |
| I-24 | " | N | 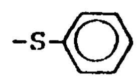 | 141-143 |
| I-25 | " | CH | 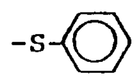 | 145 |

<u>Tabelle 1</u> (Fortsetzung)

| Verb. Nr. | Ar | Y | $R^2$ | Schmelzpunkt |
|---|---|---|---|---|
| I-26 | 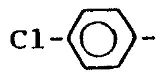 | N | 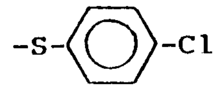 | 94 |
| I-27 | " | CH | 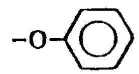 | 208 |
| I-28 | " | N | 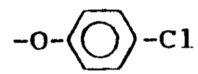 | 265 |
| I-29 | " | " | 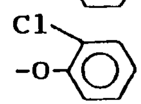 | 119 |
| I-30 | " | " | 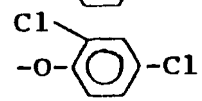 | 181 |
| I-31 | " | CH | 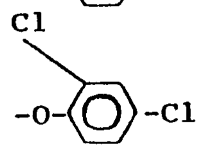 | 213 |
| I-32 | " | N | 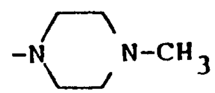 | 122 |
| I-33 | 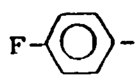 | N | F | 148 |
| I-34 | " | " | $-SCH_3$ | 113 |
| I-35 | " | CH | " | 156 |

Tabelle 1 (Fortsetzung)

| Verb. Nr. | Ar | Y | $R^2$ | Schmelzpunkt |
|---|---|---|---|---|
| I-36 | 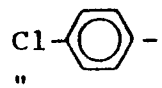 | N | $-SC_2H_5$ | 126 |
| I-37 | " | CH | " | 161 |
| I-38 | 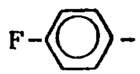 | N | $-S-(CH_2)_3CH_3$ | 130 |
| I-39 | " | CH | $-S-(CH_2)_3-CH_3$ | 140 |
| I-40 | 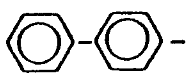 | N | 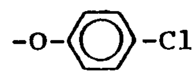 | |
| I-41 | 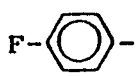 | " | 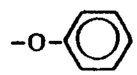 | 171 |
| I-42 | " | " | 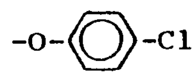 | 144 |
| I-43 | " | CH | 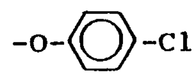 | 184 |
| I-44 | " | N | 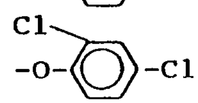 | 141 |
| I-45 | " | CH | 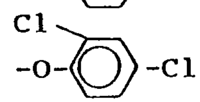 | 227 |

Tabelle 1 (Fortsetzung)

| Verb. Nr. | Ar | Y | $R^2$ | Schmelzpunkt |
|---|---|---|---|---|
| I-46 | 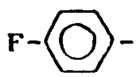 | N | 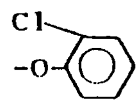 | 122 |
| I-47 | 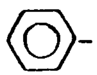 | " | $-SC_2H_5$ | 137 |
| I-48 | " | CH | " | 179 |
| I-49 | " | N | 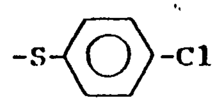 | 144 |
| I-50 | " | CH | 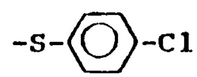 | 168 |
| I-51 | " | " | 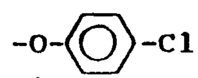 | 177 |
| I-52 | " | " | 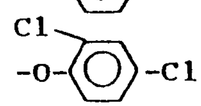 | 245 |
| I-53 | 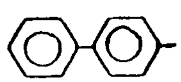 | N | F | 123 |
| I-54 | " | " | $-SCH_3$ | 163 |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-55 | 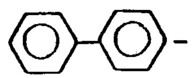 | CH | $-SCH_3$ | 160 |
| I-56 | " | N | $-SC_2H_5$ | 159 |
| I-57 | " | CH | " | |
| I-58 | " | N | $-S-(CH_2)_3-CH_3$ | 136 |
| I-59 | 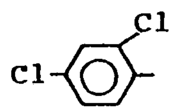 | N | $-S-C_2H_5$ | 1,5420 |
| I-60 | 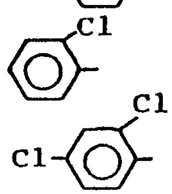 | N | $-S-C_3-H_7-n$ | 1,5102 |
| I-61 | | N | $-S-C_3-H_7-n$ | 1,5610 |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-62 | 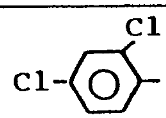 | N | $-S-C_4H_9-n$ | 86 |
| I-63 | 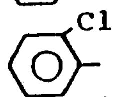 | N | $-S-C_4\ H_9-n$ | 1,5790 |
| I-64 | 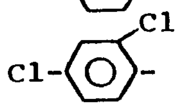 | N | $-S-CH_2-CH=C(CH_3)_2$ | 1,5698 |
| I-65 | 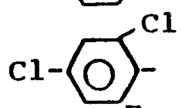 | N | $-S-C_3H_7-iso$ | 128 |
| I-66 | 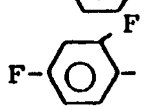 | N | $-S-C_2H_5-$ | 110 |
| I-67 | 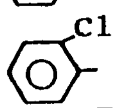 | N | $-S-CH_2-CH=C(CH_3)_2$ | 1,5598 |
| I-68 | 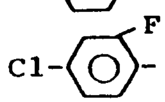 | N | $-S-C_3H_7$ | 112 |
| I-69 | 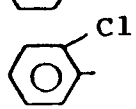 | N | $-S-C_2H_5$ | 1,5560 |
| I-70 | 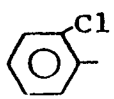 | N | $-S-CH_2-C_3H_7-iso$ | 1,5700 |

Tabelle 1 (Fortsetzung)

| Verb. Nr. | Ar | Y | $R^2$ | Schmelzpunkt /°C/ bzw. Brechungsindex /$n_D^{20}$/ |
|---|---|---|---|---|
| I-71 | 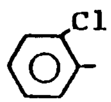 | N | $-S-(CH_2)_5-CH_3$ | 1,5430 |
| I-72 | 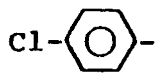 | CH | F | 176 |
| I-73 | 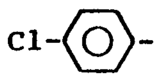 | N | 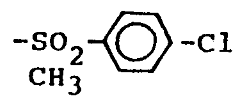 | 166 |
| I-74 | 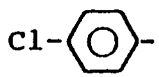 | N | $-CH(CH_3)-CH_2-CH_3$ | 104 |
| I-75 | 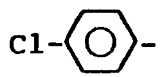 | N | $-CH(CH_3)_2$ | 137 |
| I-76 | 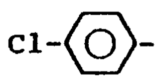 | N | $-CH_2-CH(CH_3)-CH_3$ | 147 |
| I-77 | 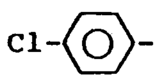 | N | $-(CH_2)_5-CH_3$ | 130 |
| I-78 | 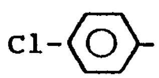 | N | $-CH_2-CH_2-CH(CH_3)-CH_3$ | 141 |
| I-79 | 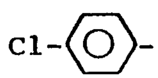 | N | $-SO-C_4H_9-n$ | Öl |

<u>Tabelle 1</u> (Fortsetzung)

| Verb. Nr. | Ar | $R^1$ | Y | $R^2$ | Schmelzpunkt [°C] bzw. Brechungsindex [$n_D^{20}$] |
|---|---|---|---|---|---|
| I-80 | 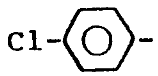 | H | N | $-SO-C_3H_7-n$ | 146 |
| I-81 | 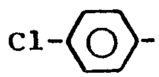 | H | N | $-SO-CH_2-CH(CH_3)-CH_3$ | 113 |
| I-82 | 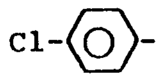 | H | N | 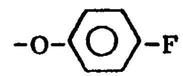 | 147 |
| I-83 | 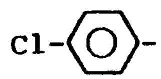 | H | N | 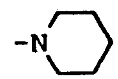 | 130 |
| I-84 | 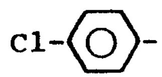 | H | N | -Cl | 149 |
| I-85 | 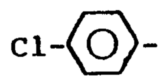 | H | N | $-S-(CH_2)_{11}-CH_3$ | 111 |
| I-86 | 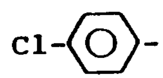 | H | N | $-SO(CH_2)_3-CH_3$ | 153 |
| I-87 | 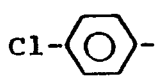 | H | CH | 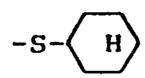 | 191 |
| I-88 | 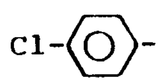 | H | N | 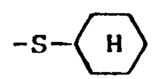 | 142 |

<u>Tabelle 1</u> (Fortsetzung)

| Verb. Nr. | Ar | Y | $R^2$ | Schmelzpunkt /°C/ bzw. Brechungsindex /$n_D^{20}$/ |
|---|---|---|---|---|
| I-89 | 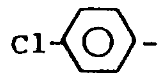 | N | $-SO-(CH_2)_{11}-CH_3$ | Öl |
| I-90 | 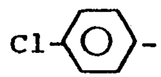 | N | $-SO_2-(CH_2)_{11}-CH_3$ | 91 |
| I-91 | 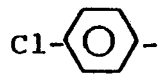 | N | $-S-CH_2-CH_2-O-$ 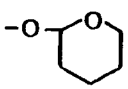 | 75 |
| I-92 | 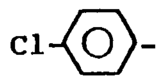 | N | $-SO-CH_3$ | 189 (1 Diastereomeres) |
| I-93 | 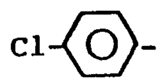 | N | $-SO-CH_3$ | 172 (Diastereomeren-Gemisch) |
| I-94 | 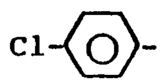 | N | $-SO_2-C_3H_7-n$ | 128 |
| I-95 | 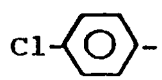 | N | $-SO_2-CH(CH_3)-C_2H_5$ | 117 |
| I-96 | 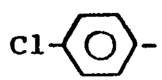 | N | $-SO_2-CH(CH_3)_2$ | 159 |
| I-97 | 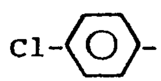 | N | $-SO_2-CH_2-CH(CH_3)_2$ | |
| I-98 | 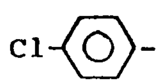 | N | $-SO_2-(CH_2)_5-CH_3$ | 140 |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-99 | Cl-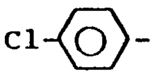- | N | $-SO_2-CH_2-CH_2-CH(CH_3)_2$ | 165 |
| I-100 | Cl-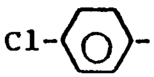- | N | $-SO_2-$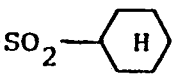 | 176 |
| I-101 | Cl-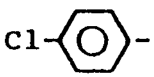- | N | $-S-CH_2-CH_2-S-C_2H_5$ | 95 |
| I-102 | Cl-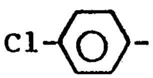- | N | $-S-CH_2-COOCH_3$ | 118 |
| I-103 | Cl-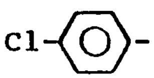- | N | $-S-CH_2-CH_2-OH$ | 138 |
| I-104 | F-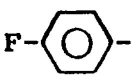- | N | $-SO-(CH_2)_3-CH_3$ | Öl |
| I-105 | F-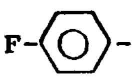- | CH | $-S-CH(CH_3)_2$ | 191 |
| I-106 | F-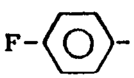- | N | $-S-CH(CH_3)_2$ | 155 |
| I-107 | F-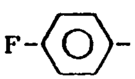- | N | -Cl | 121 |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-108 | 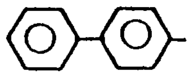 | N | $-SO_2CH_3$ | 162 |
| I-109 | 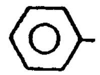 | N | $-S-CH(CH_3)_2$ | 136 |
| I-110 | 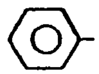 | CH | -F | 162 |
| I-111 | 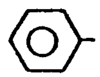 | N | -F | 105 |
| I-112 | 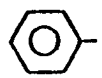 | N | $-SCH_3$ | 108 |
| I-113 | 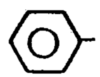 | CH | $-SCH_3$ | 167 |
| I-114 | 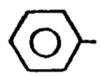 | CH | $-S-C_4H_9-n$ | 134 |
| I-115 | 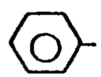 | N | $-S-C_4H_9-n$ | 125 |
| I-116 | 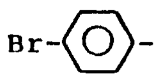 | N | -F | 123 |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-117 | 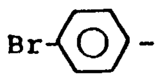 | N | $-SC_2H_5$ | 168 |
| I-118 | 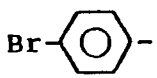 | N | -Cl | 127 |
| I-119 | 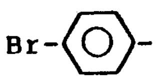 | N | $-SCH_3$ | 127 |
| I-120 | 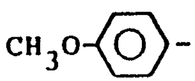 | N | -F | 111 |
| I-121 | 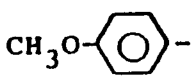 | N | $-SC_2H_5$ | 119 |
| I-122 | 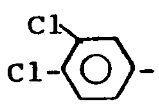 | N | 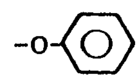 | 139 |
| I-123 | 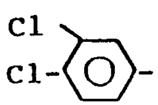 | N | $-S-C_4H_9-n$ | 170 |
| I-124 | 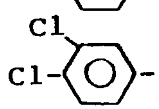 | N | $-SO-C_4H_9-n$ | Harz |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-125 | 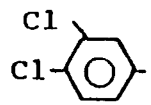 | N | $-SO_2-C_4H_9-n$ | 159 |
| I-126 | 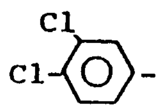 | N | $-SCH_3$ | 142 |
| I-127 | 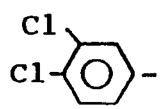 | N | $-SO_2CH_3$ | |
| I-128 | 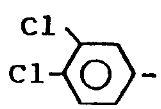 | N | $-S-CH_2-CH_2-CH_3$ | |
| I-129 | 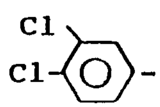 | N | $-S-C_2H_5$ | |
| I-130 | 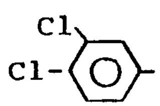 | N | $-S-CH(CH_3)_2$ | |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-131 | 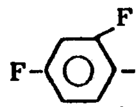 | N | $-S-CH_3$ | 83 |
| I-132 | 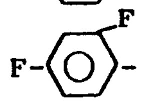 | N | $-SO_2CH_3$ | |
| I-133 | 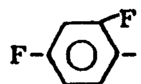 | N | -Cl | |
| I-134 | 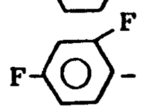 | N | 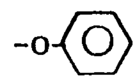 | |
| I-135 | 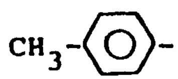 | N | -F | 127 |
| I-136 | 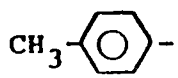 | N | $-SC_2H_5$ | 125 |
| I-137 | 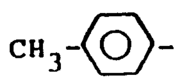 | N | 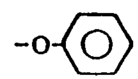 | 146 |
| I-138 | 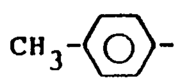 | N | $-SCH_3$ | |

<u>Tabelle 1</u> (Fortsetzung)

| Verb. Nr. | Ar | Y | $R^2$ | Schmelzpunkt [°C] bzw. Brechungsindex [$n_D^{20}$] |
|---|---|---|---|---|
| I-139 | 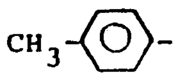 | N | $-S-CH(CH_3)_2$ | |
| I-140 | 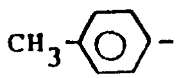 | N | $-S-CH_2-CH_2-CH_3$ | |
| I-141 | 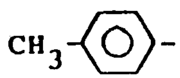 | N | $-S-CH_2-CH(CH_3)_2$ | |
| I-142 | 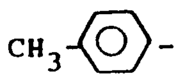 | N | $-S-(CH_2)_3-CH_3$ | |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-143 | 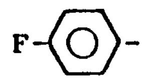 | N | $-SO_2CH_3$ | 189 |
| I-144 | 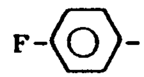 | N | $-SO_2-CH(CH_3)_2$ | 177 |
| I-145 | 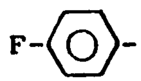 | N | $-SO-CH(CH_3)_2$ | 141 |
| I-146 | 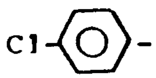 | CH | -F | 176 |
| I-147 | 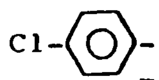 | N | $-SO-CH_3$ | 189 |
| I-148 | 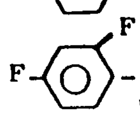 | N | -Cl | 109 |
| I-149 | 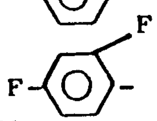 | N | 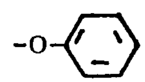 | 133 |
| I-150 | 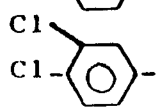 | N | $-SO_2-(CH_2)_2-CH_3$ | 104 |

<u>Tabelle 1</u> (Fortsetzung)

| Verb. Nr. | Ar | Y | $R^2$ | Schmelzpunkt [°C] |
|---|---|---|---|---|
| I-151 | 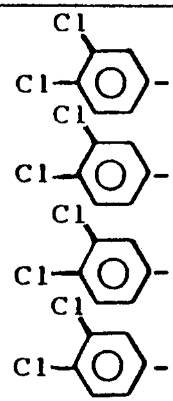 | N | $-SO-(CH_2)_2-CH_3$ | 140 |
| I-152 | | N | $-SO_2-CH_2-CH_3$ | 135 |
| I-153 | | N | $-SO_2-CH_2-CH_3$ | 112 |
| I-154 | | N | $-SO_2-CH(CH_3)_2$ | 137 |
| I-155 | 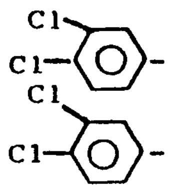 | N | $-SO-CH(CH_3)_2$ | 131 |
| I-156 | | N | $-SO_2-CH_3$ | 160 |
| I-157 | 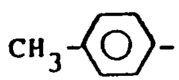 | N | $-F$ | 127 |
| I-158 | 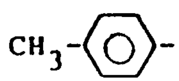 | N | $-S-CH_2-CH_3$ | 125 |
| I-159 | 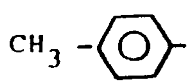 | N | $-S-CH_3$ | 116 |
| I-160 | 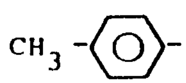 | N | $-SO_2-CH(CH_3)_2$ | 149 |

Tabelle 1 (Fortsetzung)

| Verb. Nr. | Ar | Y | $R^2$ | Schmelzpunkt [°C] |
|---|---|---|---|---|
| I-161 | 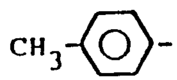 | N | $-SO-CH(CH_3)_2$ | 127 |
| I-162 | 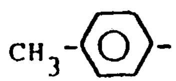 | N | $-S-(CH_2)_2-CH_3$ | 115 |
| I-163 | 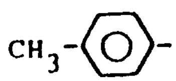 | N | $-S-CH_2-CH(CH_3)_2$ | 123 |
| I-164 | 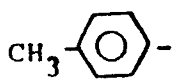 | N | $-S-(CH_2)_3-CH_3$ | 107 |
| I-165 | 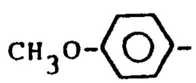 | N | -F | 111 |
| I-166 | 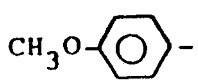 | N | $-S-CH_2-CH_3$ | 119 |

Tabelle 1 (Fortsetzung)

| Verb. Nr. | Ar | Y | $R^2$ | Schmelzpunkt [°C] bzw. Brechungsindex [$n_D^{20}$] |
|---|---|---|---|---|
| I-167 | 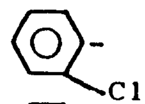 | N | -S- 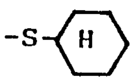 | 1,5630 |
| I-168 | 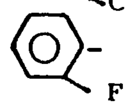 | N | $-SC_3H_7$ | 75 |
| I-169 | 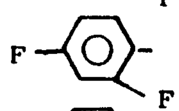 | N | $-SC_4H_9$ | 75 |
| I-170 | 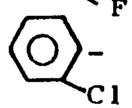 | N | $-S-CH(CH_3)_2$ | 1,5630 |
| I-171 | 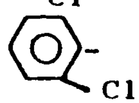 | N | $-S-C(CH_3)_3$ | 1,5660 |
| I-172 | 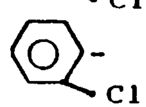 | N | $-S-CH(CH_3)-C_2H_5$ | 1,5582 |
| I-173 | 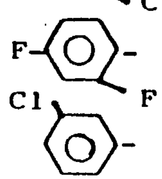 | N | $-S-CH(CH_3)_2$ | 106 |
| I-174 | | N | $-S-C_3H_7$ | 110 |
| I-175 | 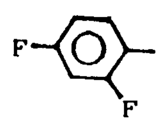 | N | $-S-C_3H_7$ | 1,5420 |

Tabelle 1 (Fortsetzung)

| Verb. Nr. | Ar | Y | $R^2$ | Schmelzpunkt [°C] |
|---|---|---|---|---|
| I-176 | 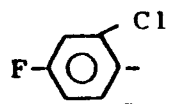 | N | $-S-CH(CH_3)_2$ | 118 |
| I-177 | 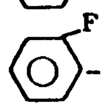 | N | $-S-CH(CH_3)_2$ | 80 |

Weitere Beispiele für die Herstellung von Ausgangsstoffen der Formel (II)

Beispiel (II—2)

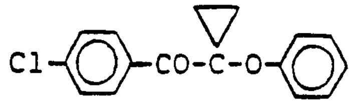

(II-2)

Zu einer Lösung von 29,6 g (0,1 Mol) 4-Chlorphenyl-(1-brom-3-chlorpropyl)-keton und 9,4 g Phenol in 60 ml Dimethylformamid gibt man 14 g Kaliumcarbonat hinzu und erwärmt drei Stunden auf 50°C. Danach tropft man bei Raumtemperatur 8,4 g Kaliumhydroxid in 40 ml Methanol und erwärmt drei Stunden auf 60°C. Man engt im Vakuum ein und nimmt in einem Wasser/Methylenchlorid-Gemisch auf. Die organische Phase wird abgetrennt, mit Wasser gewaschen, über Natriumsulfat getrocknet und im Vakuum eingeengt. Der Rückstand wird im Hochvakuum destilliert. Man erhält 20,7 g (76,1% der Theorie) 1-(4-Chlorbenzoyl)-1-phenoxy-cyclopropan vom Siedepunkt $Kp_{0,15}$ = 170—80°C.

Beispiel (II—3)

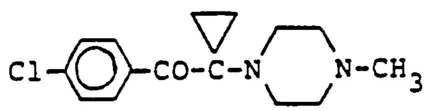

(II-3)

Zu einer Mischung aus 10,4 g N-Methylpiperazin und 14 g Kaliumcarbonat in 30 ml Dimethylformamid tropft man eine Lösung von 29,6 g (0,1 Mol) 4-Chlorphenyl-(1-brom-3-chlorpropyl)-keton in 30 ml Dimethylformamid bei Raumtemperatur ein. Man läßt drei Stunden nachrühren und tropft dann eine Lösung von 9 g Kaliumhydroxid in 30 ml Methanol zu. Das Reaktionsgemisch wird eine Stunde bei 50°C nachgerührt und im Vakuum eingeengt. Der Rückstand wird in Essigester/Wasser aufgenommen, die organische Phase abgetrennt, mit Wasser gewaschen, über Natriumsulfat getrocknet und im Vakuum eingeengt. Der Rückstand wird über Kieselgel mit Chloroform/Ethanol (97:3) chromatographiert. Man erhält 14 g (50,3% der Theorie) 1-(4-Chlorbenzoyl)-1-(4-methyl-piperazin-1-yl)-cyclopropan als rötliches Öl, das direkt weiter eingesetzt werden kann.

Beispiel (II—4)

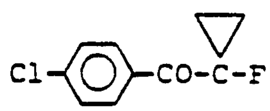

(II-4)

Man löst 20 g (0,085 Mol) 4-Chlorphenyl-(1-fluor-3-chlor-propyl)-keton in 150 ml tert.-Butanol und versetzt portionsweise mit 15 g Kalium-tert.-butylat. Man läßt 2 Stunden bei 40°C nachrühren und engt im Vakuum ein. Der Rückstand wird in Methylenchlorid und Wasser aufgenommen. Man trennt die organische Phase ab, trocknet über Natriumsulfat und engt im Vakuum ein. Der Rückstand wird im Hochvakuum destilliert. Man erhält 14,4 g (85% der Theorie) 1-(4-Chlorbenzoyl)-1-fluor-cyclopropan vom Siedepunkt $Kp_{0,1}$ = 75°C.

Herstellung von Ausgangsprodukten der Formel (VIII)

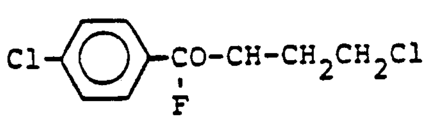

(VIII-1)

Eine Mischung aus 68 g 4-Chlorphenyl-(1-brom-3-chlorpropyl)-keton, 27,5 g getrocknetem Kaliumfluorid, 11 g 18-Krone-6 und 200 ml trockenem Benzol wird 8 Stunden unter Rückfluß erhitzt. Danach gibt man 200 ml Wasser hinzu, trennt die organische Phase ab, wäscht mehrmals mit Wasser, trocknet über Natriumsulfat und engt im Vakuum ein. Der Rückstand wird mit Leichtbenzin unter Zusatz von wenig Toluol verrührt. Nach Absaugen des anfallenden Feststoffes und Trocknen erhält man 28,5 g (53% der Theorie) 4-Chlorphenyl-(1-fluor-3-chlorpropyl)keton vom Schmelzpunkt 53°C.

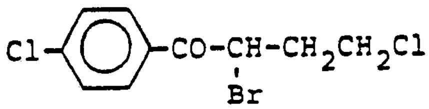

(VIII-2)

Eine Lösung von 483 g (2,23 Mol) 4-Chlorphenyl-3-chlorpropyl-keton in 1200 ml Methylenchlorid wird bei 20°C tropfenweise mit einer Lösung von 358 g Brom in 350 ml Methylenchlorid versetzt. Man läßt eine Stunde nachrühren und engt dann im Vakuum ein. Der Rückstand wird mit 300 ml Petrolether versetzt und bis zur Vollständigkeit der Kristallisation gerührt. Man kühlt auf 10°C ab und saugt den Niederschlag ab. Man erhält 582 g (88,3% der Theorie) 4-Chlorphenyl-(1-brom-3-chlorpropyl)-keton vom Schmelzpunkt 73°C.

Analog zu den in den Beispielen (II—1) bis (II—4) beschriebenen Methoden und nach den angegebenen Verfahrensbedingungen werden auch die in der folgenden Tabelle 2 aufgeführten Vorprodukte der Formel (II) hergestellt.

Tabelle 2

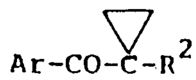 (II)

| Bsp.Nr. | Ar | $R^2$ | Physikal. Konstante |
|---|---|---|---|
| II-5 | 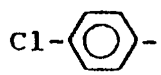 | $-S-(CH_2)_3CH_3$ | Kp.$_{0,1}$ = 133°C |
| II-6 | 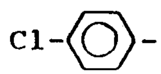 | $-S-CH_3$ | Kp.$_{0,01}$ = 120°C |
| II-8 | 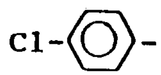 | 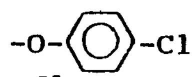 | Kp.$_{0,1}$ = 160°C |
| II-9 | 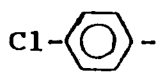 | 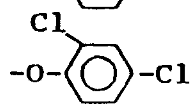 | Kp.$_{0,1}$ = 173°C |
| II-10 | 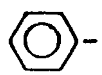 | 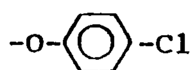 | Kp.$_{0,01}$ = 143°C |
| II-11 | 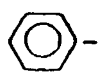 | 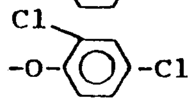 | Kp.$_{0,01}$ = 146°C |
| II-12 | 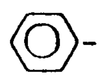 | 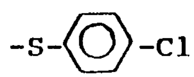 | Fp. = 86°C |
| II-13 | 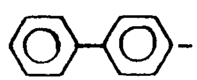 | $-SCH_3$ | Fp. = 98°C |

Tabelle 2 (Fortsetzung)

| Bsp.Nr. | Ar | $R^2$ | Physikal. Konstante |
|---|---|---|---|
| II-14 | 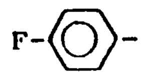 | $-SCH_3$ | $Kp._{0,1}$ = 102°C |
| II-15 | 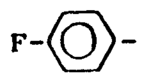 | 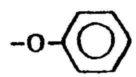 | $Kp._{0,1}$ = 125-130°C |
| II-16 | 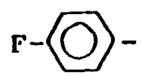 | 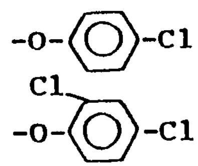 | $Kp._{0,1}$ = 145-150°C |
| II-17 | 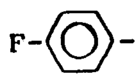 | | $Kp._{0,1}$ = 165-173°C |
| II-18 | 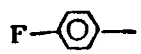 | $-S-C_2H_5$ | $Kp._{0,07}$ = 95°C |
| II-19 | 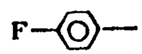 | $-S-(CH_2)_3CH_3$ | $Kp._{0,1}$ = 113-116°C |
| II-20 | 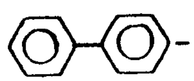 | $-S-C_2H_5$ | Fp. = 86°C |
| II-21 | 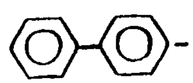 | 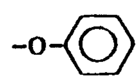 | Fp. = 77°C |
| II-22 | 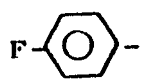 | 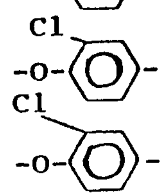 | $Kp._{0,1}$ = 147-155°C |
| II-23 | 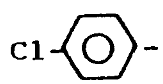 | | $Kp._{0,07}$ = 154°C |

<u>Tabelle 2</u> (Fortsetzung)

| Bsp.Nr. | Ar | $R^2$ | Physikal. Konstante |
|---|---|---|---|
| II-24 | 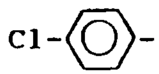 | 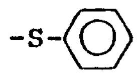 | $Kp._{0,1}$ = 163-170°C |
| II-25 | 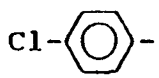 | 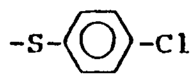 | Fp. 104°C |
| II-26 | 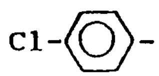 | $-S-CH_2-CH=CH_2$ | $Kp._{0,06}$ = 122°C |
| II-28 | 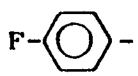 | F | $Kp._{0,07}$ = 55°C |
| II-29 | 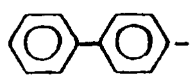 | F | $Kp._{0,15}$ = 138°C/Fp. 73°C |
| II-30 | 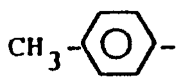 | F | $Kp._{0,1}$ = 69°C |
| II-31 | 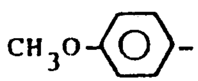 | F | $Kp._{0,1}$ = 98°C |
| II-32 | 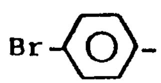 | F | $Kp._{0,1}$ = 88°C |

Nach der im Beispiel 1 beschriebenen Methode und nach den angegebenen Verfahrensbedingungen werden auch die in der folgenden Tabelle 3 aufgeführten Zwischenprodukte der Formel (IV) hergestellt.

Tabelle 3

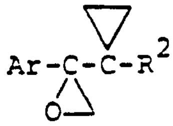

(IV)

| Bsp.Nr. | Ar | $R^2$ | Physikal. Konstante |
|---|---|---|---|
| IV-2 | 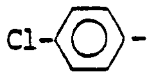 | 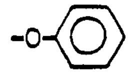 | zähfl. Öl |
| IV-3 | 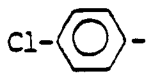 | $-S-(CH_2)_3CH_3$ | " |
| IV-4 | 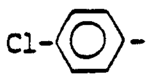 | F | $Kp._{0,2}$ = 83°C |
| IV-5 | 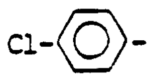 | $-SCH_3$ | Öl |
| IV-6 | 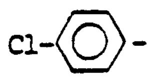 | $-SC_2H_5$ | Öl |
| IV-7 | 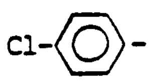 | $-S-(CH_2)_3-CH_3$ | Öl |
| IV-8 | 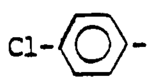 | $-S-CH_2-CH=CH_2$ | Öl |
| IV-9 | 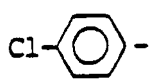 | $-S-(CH_2)_2-CH_3$ | Öl |
| IV-10 | 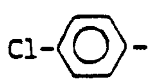 | 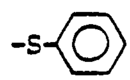 | Öl |

Tabelle 3 (Fortsetzung)

| Bsp.Nr. | Ar | $R^2$ | Physikal. Konstante |
|---|---|---|---|
| IV-11 | 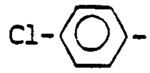 | 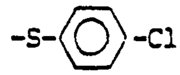 | Öl |
| IV-12 | 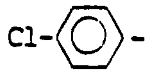 | 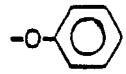 | Öl |
| IV-13 | 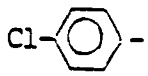 | 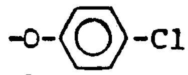 | Öl |
| IV-14 | 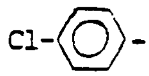 | 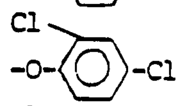 | Fp. 81°C |
| IV-15 | 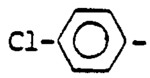 | 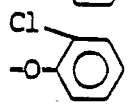 | Fp. 97°C |
| IV-16 | 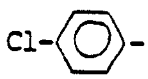 | F | $Kp._{0,2}$ = 83°C |
| IV-17 | 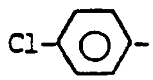 | 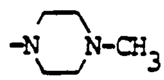 | Öl |
| IV-18 | 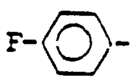 | $-SCH_3$ | Öl |
| IV-19 | 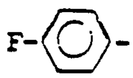 | $-SC_2H_5$ | Öl |
| IV-20 | 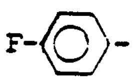 | $-S-(CH_2)_3-CH_3$ | Öl |
| IV-21 | 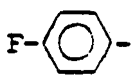 | 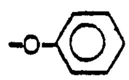 | Öl |
| IV-22 | 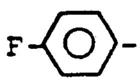 | 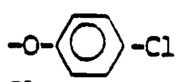 | Öl |
| IV-23 | 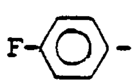 | 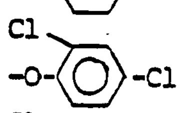 | Fp 72°C |
| IV-24 | 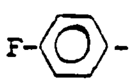 | 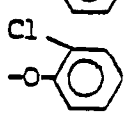 | Öl |
| IV-25 | 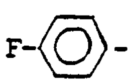 | F | Öl |

Tabelle 3 (Fortsetzung)

| Bsp.Nr. | Ar | $R^2$ | physikal. Konstante |
|---|---|---|---|
| IV-26 | 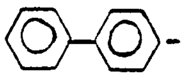 | $-SCH_3$ | Öl |
| IV-27 | 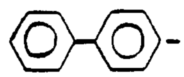 | $-SC_2H_5$ | Öl |
| IV-28 | 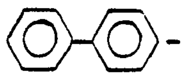 | $-S-(CH_2)_3-CH_3$ | Öl |
| IV-29 | 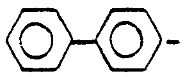 | 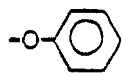 | Fp. 94°C |
| IV-30 | 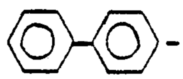 | 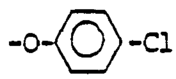 | Fp. 91°C |
| IV-31 | 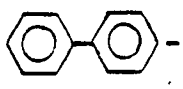 | F | Öl |
| IV-32 | 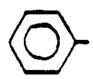 | $-SC_2H_5$ | Öl |
| IV-33 | 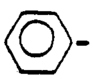 | 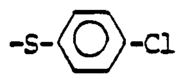 | Öl |
| IV-34 | 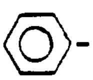 | 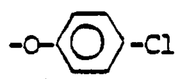 | Öl |
| IV-35 | 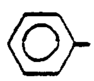 | 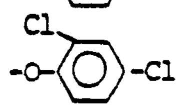 | Fp. 62°C |

Entsprechend Beispiel 2 und gemäß den angegebenen Verfahrensbedingungen werden die in der nachfolgenden Tabelle 4 aufgeführten Zwischenprodukte der Formel (VI) erhalten:

Tabelle 4

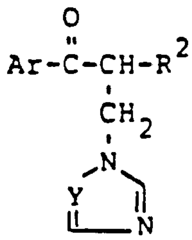 (VI)

| Bsp.Nr. | Ar | Y | $R^2$ | physikalische Konstante |
|---|---|---|---|---|
| VI-2 | 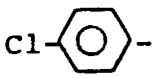 | N | 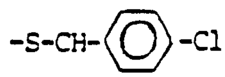 | Fp. 102°C |
| VI-3 | 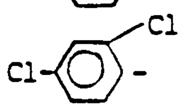 | N | 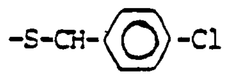 | Fp. 69°C |
| VI 4 | 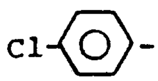 | N | 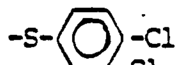 | Fp. 102°C |
| VI-5 | 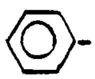 | N | 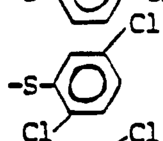 | Fp. 117°C |
| VI-6 | 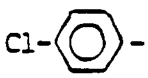 | N | 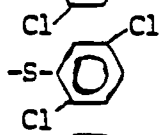 | Fp. 148°C |
| VI-7 | 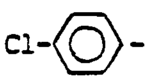 | CH | 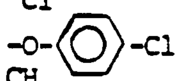 | Fp. 153°C |
| VI-8 | 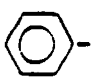 | N | 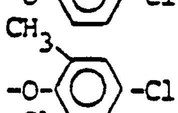 | Fp. 139°C |
| VI-9 | 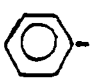 | N | 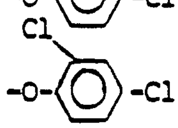 | Fp. 156°C |
| VI-10 | 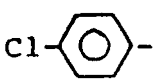 | N | 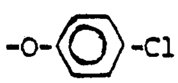 | Fp. 128°C |

Tabelle 4 (Fortsetzung)

| Bsp.Nr. | Ar | Y | $R^2$ | Schmelzpunkt (°C) Brechungsindex ($n_D^{20}$) |
|---|---|---|---|---|
| VI-11 | 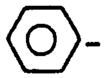 | N | 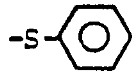 | 102 |
| VI-12 | 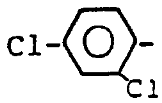 | N | $-S-C_2H_5$ | 50 |
| VI-13 | 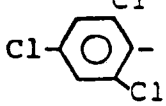 | N | $-S-C_3H_7$ | 1,5632 |
| VI-14 | 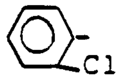 | N | $-S-C_2H_5$ | 62 |
| VI-15 | 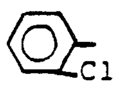 | N | $-S-C_3H_7$ | 58 |
| VI-16 | 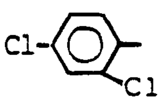 | N | $-S-C_4H_9$ | 1,5742 |
| VI-17 | 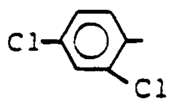 | N | $-S-CH(CH_3)_2$ | 1,5762 |

Verwendungsbeispiele

In den nachfolgenden Verwendungsbeispielen werden die nachstehend angegebenen Verbindungen als Vergleichssubstanzen eingesetzt:

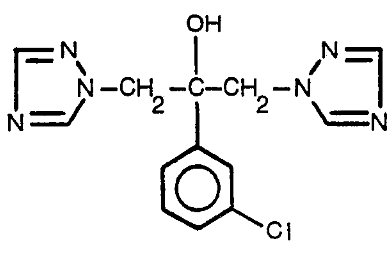

(A)

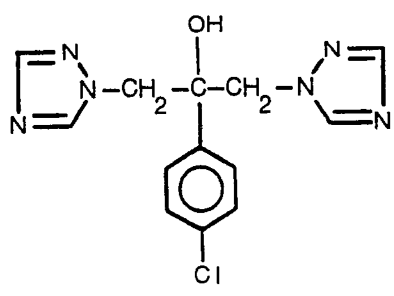

(B)

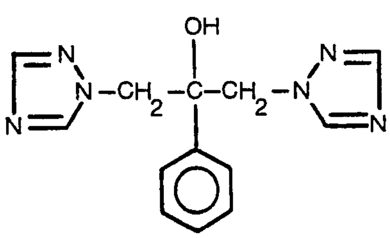

(C)

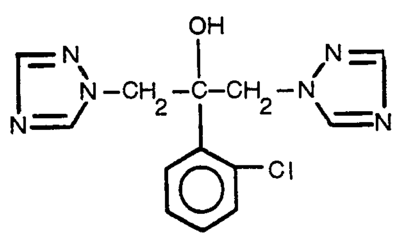

(D)

(bekannt aus EP—A 0 044 605)

Beispiel A

Wuchshemmung bei Reis

Lösungsmittel: 30 Gew.-Teile Dimethylformamid

Emulgator: 1 Gew.-Teil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Reis wird in Klimakammern in kleinen Töpfen mit Vermiculit biz zu einer Größe des 1. Blattes von 1—2 cm angezogen. In diesem Stadium werden die Töpfe bis zu einer Höhe, die der halben Höhe des Topfes entspricht, in die zubereiteten Wirkstofflösungen gestellt.

Nach der Entwicklung des 3. Blattes wird die Länge aller Pflanzen bestimmt und in Prozent der Länge der Kontrollpflanzen berechnet. Es bedeuten 100% ein Wachstum wie bei den Kontrollpflanzen, Werte unter 100% eine Wuchshemmung und Werte über 100% eine Wuchsförderung.

In diesem Test zeigen die erfindungsgemäßen Wirkstoffe (I—1), (I—6), (I—3) und (I—5) eine starke Wuchshemmung.

Beispiel B

Wuchshemmung bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigen die erfindungsgemäßen Wirkstoffe (I—4), (I—3) und (I—5) eine stärkere Wuchshemmung als die aus dem Stand der Technik bekannte Verbindung (A).

Beispiel C

Wuchshemmung bei Sojabohnen

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigen die erfindungsgemäßen Wirkstoffe (I—4) und (I—3) eine stärkere Wuchshemmung als die aus dem Stand der Technik bekannte Verbindung (D).

Beispiel D

Wuchshemmung bei Gerste

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigen die erfindungsgemäßen Wirkstoffe (I—4), (I—3) und (I—5) eine stärkere Wuchshemmung als die aus dem Stand der Technik bekannten Verbindungen (A), (B), (C) und (D).

Beispiel E

Venturia-Test (Apfel)/protektiv/

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

In diesem Test zeigt die erfindungsgemäße Verbindung (I—1) eine bessere Wirksamkeit als die bekannte Vergleichssubstanz (C).

**Patentansprüche**

1. Substituierte Azolylmethyl-cyclopropyl-carbinol-Derivate der Formel

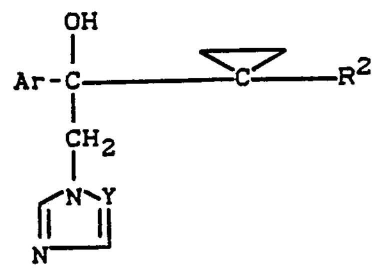

(I)

in welcher

Ar für Phenyl steht, das einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

Ar für Naphthyl steht,

$R^2$ für Fluor, Chlor, Brom oder für die Gruppierungen —X—$R^3$ und —$NR^4R^5$ steht, worin

$R^3$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 8 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie für Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl oder Phenoxy, oder

$R^3$ für den Rest der Formel

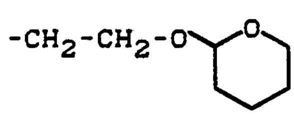

steht,

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann, und

X für Sauerstoff, Schwefel, eine SO— oder eine $SO_2$-Gruppe steht, und

Y für Stickstoff oder eine CH-Gruppe steht,

sowie deren Säureadditions-Salze und Metallsalz-Komplexe.

2. Verfahren zur Herstellung von substituierten Azolylmethyl-cyclopropyl-carbinol-Derivaten der Formel

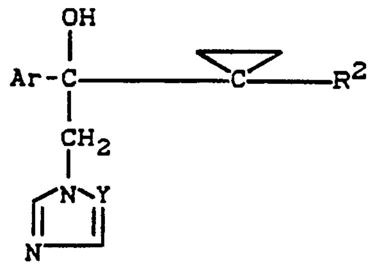

(I)

in welcher

Ar für Phenyl steht, das einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

Ar für Naphthyl steht,

$R^2$ für Fluor, Chlor, Brom oder für die Gruppierungen $-X-R^3$ und $-NR^4R^5$ steht, worin

$R^3$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 8 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie für Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl oder Phenoxy, oder

$R^3$ für den Rest der Formel

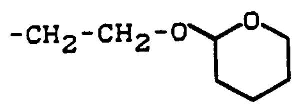 steht,

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann, und

X für Sauerstoff, Schwefel, eine SO— oder eine $SO_2$-Gruppe steht, und

Y für Stickstoff oder eine CH-Gruppe steht,

sowie deren Säureadditions-Salze und Metallsalz-Komplexen, dadurch gekennzeichnet, daß man

a) in einer *ersten Stufe* Aryl-cyclopropyl-ketone der Formel

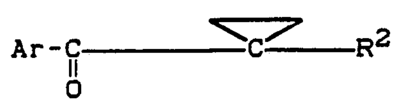

(II)

in welcher Ar und $R^2$ die oben angegebene Bedeutung haben,
mit Dimethyloxosulfonium-methylid der Formel

$$(CH_3)_2\ {}^{\delta+}SO^{\delta-}CH_2 \qquad (III)$$

in Gegenwert eines Verdünnungsmittels umsetzt und die dabei entstehenden Aryl-cyclopropyloxirane der Formel

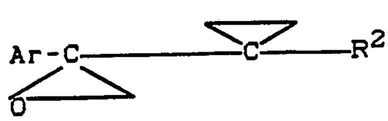

(IV)

in welcher Ar und $R^2$ die oben angegebene Bedeutung haben,
in einer *zweiten Stufe* mit Azolen der Formel

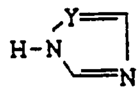

(V)

in welcher Y die oben angegebene Bedeutung hat,
in Gegenwart eines Verdünnungsmittels und in Gegenwart einer Base umsetzt, oder

b) Azolyl-keto Verbindungen der Formel

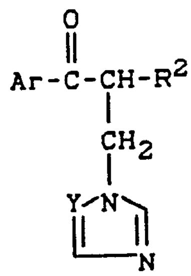

(VI)

in welcher $R^2$, Ar und Y die oben angegebene Bedeutung haben
mit Dimethyloxosulfonium-methylid der Formel

$$(CH_3)_2 \, ^{\delta+}SO^{\delta-}CH_2 \qquad (III)$$

in Gegenwert eines Verdünnungsmittels umsetzt oder

c) Azolylmethyl-thio-cyclopropyl-carbinol-Derivate der Formel

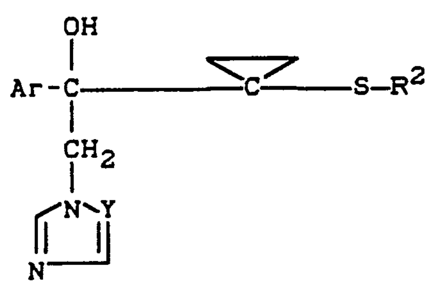

(Ia)

in welcher
Ar, $R^3$ und Y die oben angegebene Bedeutung haben,
mit Oxidationsmitteln gegebenenfalls in Gegenwart eines Verdünnungsmittels umsetzt,
und gegebenenalls an die so erhaltenen Verbindungen der Formel (I) eine Säure oder ein Metallsalz addiert.

3. Pflanzenwachstumregulierende und fungizide Mittel, gekennzeichnet durch einen Gehalt an mindestens einem substituierten Azolylmethyl-cyclopropyl-carbinol Derivat der Formel (I) gemäß Anspruch 1 bzw. einem Säureadditions-Salz oder Metallsalz-Komplex eines substituierten Azolylmethyl-cyclopropyl-carbinol-Derivates der Formel (I).

4. Verwendung von substituierten Azolylmethyl-cyclopropyl-carbinol-Derivaten der Formel (I) gemäß Anspruch 1 bzw. von deren Säureadditions-Salzen und Metallsalz-Komplexen zur Regulierung des Pflanzenwachstums und zur Bekämptfung phytopathogener Pilze.

5. Aryl-cyclopropyl-ketone der Formel

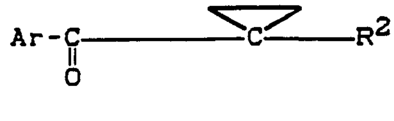

(IIa)

in welcher
Ar für Phenyl steht, das einfach oder mehrfach, gleichartig oder verschieden substituiert sein kann

durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

Ar für Naphthyl steht,

$R^2$ für Fluor, Chlor, Brom oder für die Gruppierungen —X'—$R^3$ und —$NR^4R^5$ steht, worin

$R^3$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 8 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie für Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl oder Phenoxy, oder

$R^3$ für den Rest der Formel

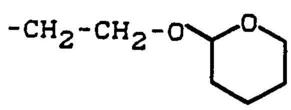 steht,

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann, und

X' für Sauerstoff, eine SO— oder eine $SO_2$-Gruppe steht.

6. Verfahren zur Herstellung Aryl-cyclopropylketonen der Formel

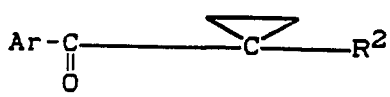 (IIa)

in welcher

Ar für Phenyl steht, das einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

Ar für Naphthyl steht,

$R^2$ für Fluor, Chlor, Brom oder für die Gruppierungen —X'—$R^3$ und —$NR^4R^5$ steht, worin

$R^3$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 8 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie für Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl oder Phenoxy, oder

$R^3$ für den Rest der Formel

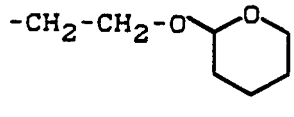 steht,

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann, und

X' für Sauerstoff, eine SO— oder eine $SO_2$-Gruppe steht.

dadurch gekennzeichnet, daß man Arylhalolgenpropylketone der Formel

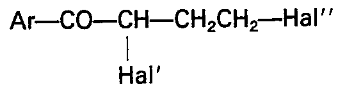

(VIII)

in welcher

Ar die oben angegebene Bedeutung hat,

Hal' für Fluor, Chlor oder Brom steht und

Hal'' für Brom oder Chlor steht,

α) mit Verbindungen der Formel

$$HR^6 \quad (IX)$$

in welcher

$R^6$ für die Gruppierungen $—X'R^3$ und $—NR^4R^5$ steht, worin $R^3$, $R^4$, $R^5$ und X' die oben angegebenen Bedeutungen haben,

in Gegenwart eines Verdünnungsmittels und in Gegenwart einer Base umsetzt,

β) direkt in Gegenwart eines Verdünnungsmittels und in Gegenwart einer Base erhitzt.

7. Aryl-cyclopropyl-oxirane der Formel

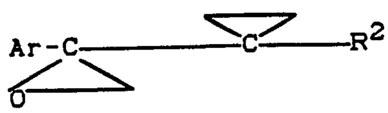

(IV)

in welcher

Ar für Phenyl steht, das einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

Ar für Naphthyl steht,

$R^2$ für Fluor, Chlor, Brom oder für die Gruppierungen $—X—R^3$ und $—NR^4R^5$ steht, worin

$R^3$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 8 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie für Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl oder Phenoxy, oder

$R^3$ für den Rest der Formel

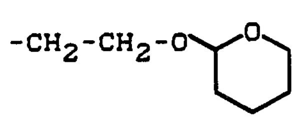

steht,

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann, und

X für Sauerstoff, Schwefel, eine SO— oder eine $SO_2$-Gruppe steht.

8. Verfahren zur Herstellung von Aryl-cyclopropyl-oxiranen der Formel

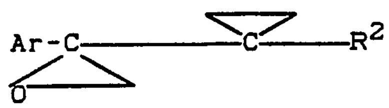

(IV)

in welcher

Ar für Phenyl steht, das einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl und Phenoxy, oder

Ar für Naphthyl steht,

$R^2$ für Fluor, Chlor, Brom oder für die Gruppierungen —X—$R^3$ und —$NR^4R^5$ steht, worin

$R^3$ für geradkettiges oder verzweigtes Alkyl mit 1 bis 18 Kohlenstoffatomen, Cycloalkyl mit 3 bis 8 Kohlenstoffatomen, geradkettiges oder verzweigtes Alkenyl mit 2 bis 8 Kohlenstoffatomen, geradkettiges oder verzweigtes Alkinyl mit 2 bis 18 Kohlenstoffatomen, Hydroxyalkyl mit 1 bis 18 Kohlenstoffatomen, Alkylthioalkyl mit 1 bis 6 Kohlenstoffatomen in der Alkylthiogruppe und 1 bis 6 Kohlenstoffatomen in der Alkylgruppe, Carboxyalkyl mit 1 bis 18 Kohlenstoffatomen im Alkylteil, Alkoxycarbonylalkyl mit 1 bis 6 Kohlenstoffatomen im Alkoxyteil und 1 bis 6 Kohlenstoffatomen im Alkylteil, sowie für Phenyl oder Phenylalkyl mit 1 oder 2 Kohlenstoffatomen im Alkylteil steht, wobei jeder der Phenyl-Reste einfach oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Phenyl oder Phenoxy, oder

$R^3$ für den Rest der Formel

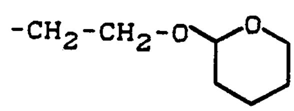 steht,

$R^4$ und $R^5$ unabhängig voneinander für Wasserstoff oder geradkettiges oder verzweigtes Alkyl mit 1 bis 4 Kohlenstoffatomen stehen oder

$R^4$ und $R^5$ gemeinsam mit dem Stickstoffatom, an das sie gebunden sind, für einen gegebenenfalls durch Alkyl mit 1 bis 4 Kohlenstoffatomen substituierten 5- oder 6-gliedrigen Ring stehen, der Sauerstoff, Schwefel und/oder Stickstoff als weitere Heteroatome enthalten kann, und

X für Sauerstoff, Schwefel, eine SO— oder eine $SO_2$-Gruppe steht,

dadurch gekennzeichnet, daß man Arylcyclopropyl-ketone der Formel

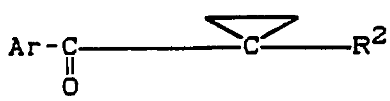

(II)

in welcher

Ar und $R^2$ die oben angegebene Bedeutung haben,

mit Dimethyloxosulfonium-methylid der Formel

$$(CH_3)_2\ {}^{\delta+}SO^{\delta-}CH_2 \qquad (III)$$

in Gegenwart eines Verdünnungsmittels umsetzt.

**Revendications**

1. Dérivés substitués d'azolylméthyl-cyclopropyl-carbinol de formule

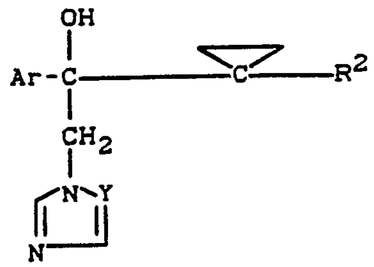

(I)

dans laquelle

Ar est un groupe phényle qui peut porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle et phénoxy, ou bien

Ar est un groupe naphtyle,

$R^2$ désigne le fluor, le chlore, le brome ou les groupements $-X-R^3$ et $-NR^4R^5$, où

$R^3$ est un groupe alkyle à chaîne droite ou à chaîne ramifée ayant 1 à 18 atomes de carbone, un groupe cycloalkyle ayant 3 à 8 atomes de carbone, un groupe alcényle à chaîne droite ou à chaîne ramifiée ayant 2 à 18 atomes de carbone, un groupe alcynyle à chaîne droite ou à chaîne ramifiée ayant 2 à 18 atomes de carbone, un groupe hydroxyalkyle ayant 1 à 18 atomes de carbone, un groupe alkylthioalkyle ayant 1 à 6 atomes de carbone dans le groupe alkylthio et 1 à 6 atomes de carbone dans le groupe alkyle, un groupe carboxyalkyle ayant 1 à 18 atomes de carbone dans la partie alkyle, un groupe alkoxycarbonylalkyle ayant 1 à 6 atomes de carbone dans la partie alkoxy et 1 à 6 atomes de carbone dans la partie alkyle, ainsi qu'un groupe phényle ou phénylalkyle ayant 1 ou 2 atomes de carbone dans la partie alkyle, chacun des restes phényle pouvant porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, un groupe phényle ou phénoxy ou bien

$R^3$ représente le reste de formule

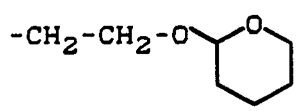

$R^4$ et $R^5$ représentent, indépendamment l'un de l'autre, l'hydrogène ou un groupe alkyle à chaîne droite ou à chaîne ramifiée ayant 1 à 4 atomes de carbone, ou bien

$R^4$ et $R^5$ forment, conjointement avec l'atome d'azote auquel ils sont liés, un noyau pentagonal ou hexagonal éventuellement substitué par un radical alkyle ayant 1 à 4 atomes de carbone et pouvant contenir de l'oxygène, du soufre et/ou de l'azote comme autres hétéroatomes, et

X représente l'oxygène, le soufre, un groupe SO— ou un groupe $SO_2$, et

Y représente de l'azote ou un groupe CH,

ainsi que leurs sels d'addition d'acides et leurs complexes de sels métalliques.

2. Procédé de production de dérivés substitués d'azolylméthylcyclopropylcarbonyl de formule

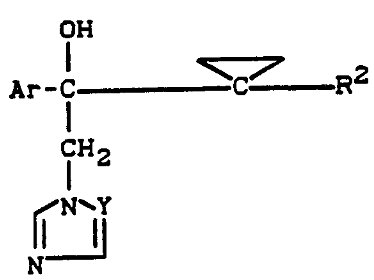

(I)

dans laquelle

Ar est un groupe phényle qui peut porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle et phénoxy, ou bien

Ar est un groupe naphtyle,

$R^2$ désigne le fluor, le chlore, le brome ou les groupements —X—$R^3$ et —$NR^4R^5$, où

$R^3$ est un groupe alkyle à chaîne droite ou à chaîne ramifiée ayant 1 à 18 atomes de carbone, un groupe cycloalkyle ayant 3 à 8 atomes de carbone, un groupe alcényle à chaîne droite ou à chaîne ramifiée ayant 2 à 18 atomes de carbone, un groupe alcynyle à chaîne droite ou à chaîne ramifiée ayant 2 à 18 atomes de carbone, un groupe hydroxyalkyle ayant 1 à 18 atomes de carbone, un groupe alkylthioalkyle ayant 1 à 6 atomes de carbone dans le groupe alkylthio et 1 à 6 atomes de carbone dans le groupe alkyle, un groupe carboxyalkyle ayant 1 à 18 atomes de carbone dans la partie alkyle, un groupe alkoxycarbonylalkyle ayant 1 à 6 atomes de carbone dans la partie alkoxy et 1 à 6 atomes de carbone dans la partie alkyle, ainsi qu'un groupe phényle ou phénylalkyle ayant 1 ou 2 atomes de carbone dans la partie alkyle, chacun des restes phényle pouvant porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, un groupe phényle ou phénoxy ou bien

$R^3$ représente le reste de formule

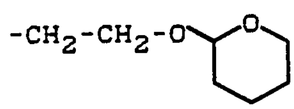

$R^4$ et $R^5$ représentent, indépendamment l'un de l'autre, l'hydrogène ou un groupe alkyle à chaîne droite ou à chaîne ramifiée ayant 1 à 4 atomes de carbone, ou bien

$R^4$ et $R^5$ forment, conjointement avec l'atome d'azote auquel ils sont liés, un noyau pentagonal ou hexagonal éventuellement substitué par un radical alkyle ayant 1 à 4 atomes de carbone et pouvant contenir de l'oxygène, du soufre et/ou de l'azote comme autres hétéroatomes, et

X représente l'oxygène, le soufre, un groupe SO— ou un groupe $SO_2$, et

Y représente de l'azote ou un groupe CH, ainsi que leurs sels d'addition d'acides et leurs complexes de sels métalliques, caractérisé en ce que

a) on fait réagir dans une *première étape* des arylcyclopropylcétones de formule

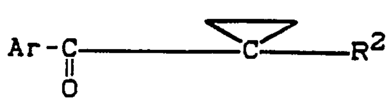

(II)

dans laquelle

Ar et $R^2$ ont la définition indiquée ci-dessus avec le méthylure de diméthoxysulfonium de formule

$$(CH_3)_2\ {}^{\delta+}SO^{\delta-}CH_2 \qquad (III)$$

en présence d'un diluant et, dans une *seconde étape,* on fait réagir les arylcyclopropyloxirannes ainsi produits de formule

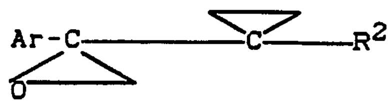

(IV)

dans laquelle

Ar et $R^2$ ont la définition indiquée ci-dessus,

avec des azoles de formule

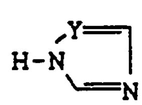

(V)

dans laquelle

Y a la définition indiquée ci-dessus,

en présence d'un diluant et en présence d'une base, ou bien

b) on fait réagir des composés azolylcétoniques de formule

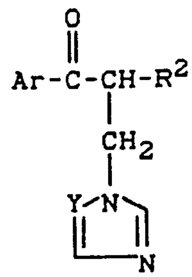

(VI)

dans laquelle

$R^2$, Ar et Y ont la définition indiquée ci-dessus,

avec le méthylure de diméthyloxosulfonium de formule

$$(CH_3)_2 \, ^{\delta+}SO^{\delta-}CH_2 \qquad (III)$$

en présence d'un diluant, ou bien

c) on fait réagir des dérivés d'azolylméthylthiocyclopropylcarbinol de formule

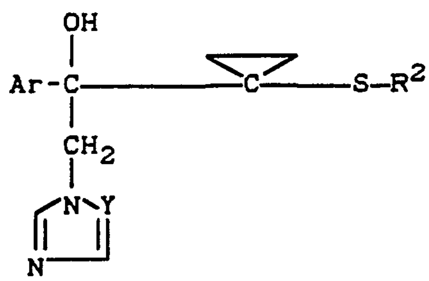

(Ia)

dans laquelle

Ar, $R^3$ et Y ont la définition indiquée ci-dessus,

avec des agents oxydants, le cas échéant en présence d'un diluant,

et on additionne le cas échéant sur les composés de formule (I) ainsi obtenus, un acide ou sel métallique.

3. Compositions régulatrices de croissance des plants et fongicides, caractérisées par une teneur en au moins un dérivé substitué d'azolylméthylcyclopropylcarbinol de formule (I) suivant la revendication 1 ou en un sel d'addition d'acide ou en un complexe de sel métallique d'un dérivé substitué d'azolylméthylcyclopropylcarbinol de formule (I).

4. Utilisation de dérivés substitués d'azolylméthylcyclopropylcarbinol de formule (I) suivant la revendication 1 ou de leurs sels d'addition d'acides et de leurs complexes de sels métalliques pour la régulations de la croissance de plantes et pour combattre des champignons phytopathogènes.

5. Arylcyclopropylcétones de formule

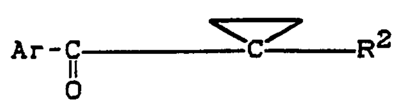

(IIa)

dans laquelle

Ar est un groupe phényle qui peut porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle et phénoxy, ou bien

Ar est un groupe naphtyle,

$R^2$ représente le fluor, le chlore, le brome ou les groupements $—X'—R^3$ et $—NR^4R^5$, dans lesquels

$R^3$ est un groupe alkyle à chaîne droite ou à chaîne ramifée ayant 1 à 18 atomes de carbone, un groupe cycloalkyle ayant 3 à 18 atomes de carbone, un groupe alcényle à chaîne droite ou à chaîne ramifiée ayant 2

à 18 atomes de carbone, un groupe alcynyle à chaîne droite ou à chaîne ramifiée ayant 2 à 18 atomes de carbone, un groupe hydroxyalkyle ayant 1 à 18 atomes de carbone, un groupe alkylthioalkyle ayant 1 à 6 atomes de carbone dans le groupe alkylthio et 1 à 6 atomes de carbone dans le groupe alkyle, un groupe carboxyalkyle ayant 1 à 18 atomes de carbone dans la partie alkyle, un groupe alkoxycarbonylalkyle ayant 1 à 6 atomes de carbone dans la partie alkoxy et 1 à 6 atomes de carbone dans la partie alkyle, ainsi qu'un groupe phényle ou phénylalkyle ayant 1 ou 2 atomes de carbone dans la partie alkyle, chacun des restes phényle pouvant porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle ou phénoxy ou bien

$R^3$ représente le reste de formule

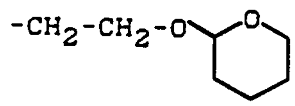

$R^4$ et $R^5$ représentent, indépendamment l'un de l'autre, l'hydrogène ou un groupe alkyle à chaîne droite ou ramifiée ayant 1 à 4 atomes de carbone, ou bien

$R^4$ et $R^5$ forment, conjointement avec l'atome d'azote auquel ils sont liés, un noyau pentagonal ou hexagonal éventuellement substitué par un radical alkyle ayant 1 à 4 atomes de carbone et qui peut contenir de l'oxygène, du soufre et/ou de l'azote comme autres hétéroatomes, et

X' représente l'oxygène, un groupe SO ou un groupe $SO_2$.

6. Procédé de préparation d'arylcyclopropylcétones de formule

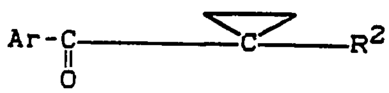

(IIa)

dans laquelle

Ar est un groupe phényle qui peut porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle et phénoxy, ou bien

Ar est un groupe naphtyle,

$R^2$ représente le fluor, le chlore, le brome ou les groupements —X'—$R^3$ et —$NR^4R^5$, dans lesquels

$R^3$ est un groupe alkyle à chaîne droite ou à chaîne ramifée ayant 1 à 18 atomes de carbone, un groupe cycloalkyle ayant 3 à 8 atomes de carbone, un groupe alcényle à chaîne droite ou à chaîne ramifiée ayant 2 à 18 atomes de carbone, un groupe alcynyle à chaîne droite ou à chaîne ramifiée ayant 2 à 18 atomes de carbone, un groupe hydroxyalkyle ayant 1 à 18 atomes de carbone, un groupe alkylthioalkyle ayant 1 à 6 atomes de carbone dans le groupe alkylthio et 1 à 6 atomes de carbone dans le groupe alkyle, un groupe carboxyalkyle ayant 1 à 18 atomes de carbone dans la partie alkyle, un groupe alkoxycarbonylalkyle ayant 1 à 6 atomes de carbone dans la partie alkoxy et 1 à 6 atomes de carbone dans la partie alkyle, ainsi qu'un groupe phényle ou phénylalkyle ayant 1 ou 2 atomes de carbone dans la partie alkyle, chacun des restes phényle pouvant porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle ou phénoxy ou bien

$R^3$ représente le reste de formule

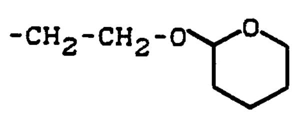

$R^4$ et $R^5$ représentent, indépendamment l'un de l'autre, l'hydrogène ou un groupe alkyle à chaîne droite ou ramifiée ayant 1 à 4 atomes de carbone, ou bien

$R^4$ et $R^5$ forment, conjointement avec l'atome d'azote auquel ils sont liés, un noyau pentagonal ou

hexagonal qui est éventuellement substitué par un radical alkyle de 1 à 4 atomes de carbone et qui peut contenir de l'oxygène, du soufre et/ou de l'azote comme autres hétéroatomes, et

X' représente l'oxygène, un groupe SO ou un groupe $SO_2$,

caractérisé en ce qu'on fait réagir des arylhalogénopropylcétones de formule

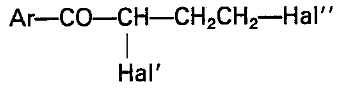

(VIII)

dans laquelle

Ar a la définition indiquée ci-dessus,

Hal' représente le fluor, le chlore ou le brome et

Hal'' représente le brome ou le chlore,

α) avec des composés de formule

$$HR^6 \qquad (IX)$$

dans laquelle

$R^6$ représente les groupements —X'$R^3$ et —$NR^4R^5$ où $R^3$, $R^4$, $R^5$ et X' ont les définitions indiquées ci-dessus,

en présence d'un diluant et en présence d'une base,

β) on les chauffe directement en présence d'un diluant et en présence d'une base.

7. Arylcyclopropyloxirannes de formule

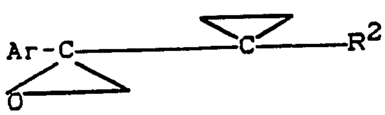

(IV)

dans laquelle

Ar est un groupe phényle qui peut porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle et phénoxy, ou bien

Ar est un groupe naphtyle,

$R^2$ représente le fluor, le chlore, le brome ou les groupements —X—$R^3$ et —$NR^4R^5$, dans lesquels

$R^3$ est un groupe alkyle à chaîne droite ou ramifiée ayant 1 à 18 atomes de carbone, cycloalkyle ayant 3 à 8 atomes de carbone, alcényle à chaîne droite ou ramifiée ayant 2 à 18 atomes de carbone, alcynyle à chaîne droite ou ramifiée ayant 2 à 18 atomes de carbone, hydroxyalkyle ayant 1 à 18 atomes de carbone, alkylthioalkyle ayant 1 à 6 atomes de carbone dans le groupe alkylthio et 1 à 6 atomes de carbone dans le groupe alkyle, carboxyalkyle ayant 1 à 18 atomes de carbone dans la partie alkyle, alkoxycarbonylalkyle ayant 1 à 6 atomes de carbone dans la partie alkoxy et 1 à 6 atomes de carbone dans la partie alkyle, ainsi qu'un groupe phényle ou phénylalkyle ayant 1 ou 2 atomes de carbone dans la partie alkyle, chacun des restes phényle pouvant porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle ou phénoxy ou bien

$R^3$ représente le reste de formule

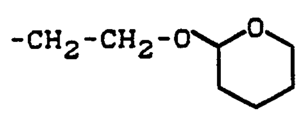

$R^4$ et $R^5$ représentent, indépendamment l'un de l'autre, l'hydrogène ou un groupe alkyle à chaîne droite ou ramifiée ayant 1 à 4 atomes de carbone, ou bien

$R^4$ et $R^5$ forment, conjointement avec l'atome d'azote auquel ils sont liés, un noyau pentagonal ou hexagonal qui porte éventuellement substituant alkyle ayant 1 à 4 atomes de carbone et qui peut contenir de l'oxygène, du soufre et/ou de l'azote comme autres hétéroatomes, et

X représente l'oxygène, le soufre, un groupe SO ou un groupe $SO_2$.

8. Procédé de production d'arylcyclopropyl oxirannes de formule

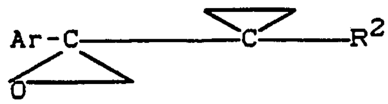 (IV)

dans laquelle

Ar est un groupe phényle qui peut porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle et phénoxy, ou bien

Ar est un groupe naphtyle,

$R^2$ représente le fluor, le chlore, le brome ou les groupements —X—$R^3$ et —$NR^4R^5$, dans lesquels

$R^3$ est un groupe alkyle à chaîne droite ou ramifiée ayant 1 à 18 atomes de carbone, cycloalkyle ayant 3 à 8 atomes de carbone, alcényle à chaîne droite ou ramifiée ayant 2 à 18 atomes de carbone, alcynyle à chaîne droite ou ramifiée ayant 2 à 18 atomes de carbone, hydroxyalkyle ayant 1 à 18 atomes de carbone, alkylthioalkyle ayant 1 à 6 atomes de carbone dans le groupe alkylthio et 1 à 6 atomes de carbone dans le groupe alkyle, carboxyalkyle ayant 1 à 18 atomes de carbone dans la partie alkyle, alkoxycarbonylalkyle ayant 1 à 6 atomes de carbone dans la partie alkoxy et 1 à 6 atomes de carbone dans la partie alkyle, ainsi qu'un groupe phényle ou phénylalkyle ayant 1 ou 2 atomes de carbone dans la partie alkyle, chacun des restes phényle pouvant porter un ou plusieurs substituants, identiques ou différents, halogène, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, phényle ou phénoxy ou bien

$R^3$ représente le reste de formule

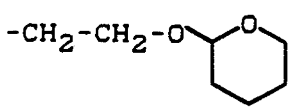

$R^4$ et $R^5$ représentent, indépendamment l'un de l'autre, l'hydrogène ou un groupe alkyle à chaîne droite ou ramifiée ayant 1 à 4 atomes de carbone, ou bien

$R^4$ et $R^5$ forment, conjointement avec l'atome d'azote auquel ils sont liés, un noyau pentagonal ou hexagonal qui porte éventuellement substituant alkyle ayant 1 à 4 atomes de carbone et qui peut contenir de l'oxygène, du soufre et/ou de l'azote comme autres hétéroatomes, et

X' représente l'oxygène, le soufre, un groupe SO ou un groupe $SO_2$,

caractérisé en ce qu'on fait réagir des arylcyclopropylcétones de formule

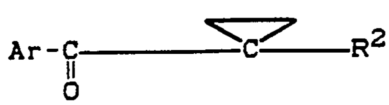 (II)

dans laquelle

Ar et $R^2$ ont la définition indiquée ci-dessus, avec le méthylure de diméthyloxosulfonium de formule

$$(CH_3)_2\ ^{\delta+}SO^{\delta-}CH_2 \qquad (III)$$

en présence d'un diluant.

**Claims**

1. Substituted azolylmethyl-cyclopropyl-carbinol derivatives of the formula

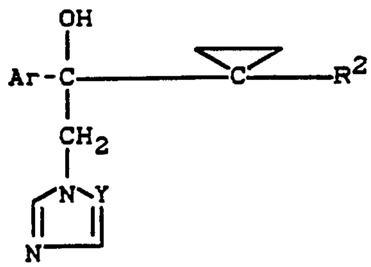

(I)

in which

Ar represents phenyl which can be monosubstituted or polysubstituted by identical or different substituents, substituents being: halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl and phenoxy, or

Ar represents naphthyl,

$R^2$ represents fluorine, chlorine, bromine or represents the groupings —X—$R^3$ and —$NR^4R^5$, wherein

$R^3$ represents straight-chain or branched alkyl having 1 to 18 carbon atoms, cycyloalkyl having 3 to 8 carbon atoms, straight-chain or branched alkenyl having 2 to 18 carbon atoms, straight-chain or branched alkinyl having 2 to 18 carbon atoms, hydroxyalkyl having 1 to 18 carbon atoms, alkylthioalkyl having 1 to 6 carbon atoms in the alkylthio group and 1 to 6 carbon atoms in the alkyl group, carboxyalkyl having 1 to 18 carbon atoms in the alkyl part, alkoxycarbonylalkyl having 1 to 6 carbon atoms in the alkoxy part and 1 to 6 carbon atoms in the alkyl part, and phenyl or phenylalkyl having 1 or 2 carbon atoms in the alkyl part, in which each of the phenyl radicals can be monosubstituted or polysubstituted by identical or different substituents, substituents being halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl or phenoxy, or

$R^3$ represents the radical of the formula

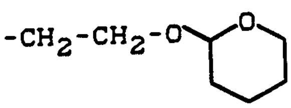

$R^4$ and $R^5$ independently of one another represent hydrogen or straight-chain or branched alkyl having 1 to 4 carbon atoms, or

$R^4$ and $R^5$, together with the nitrogen atom to which they are bonded, represent a 5-membered or 6-membered ring which is optionally substituted by alkyl having 1 to 4 carbon atoms, which can contain oxygen, sulphur and/or nitrogen as further hetero atoms, and

X represents oxygen, sulphur, an SO group or an $SO_2$ group, and

Y represents nitrogen or a CH group,

and their acid addition salts and metal salt complexes.

2. Process for the preparation of substituted azolylmethyl-cyclopropyl-carbinol derivatives of the formula

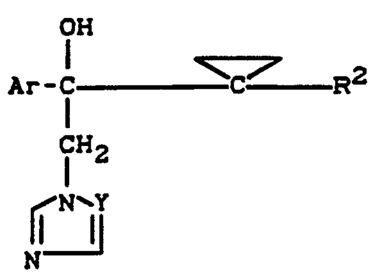

(I)

in which

Ar represents phenyl which can be monosubstituted or polysubstituted by identical or different substituents, substituents being: halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl and phenoxy, or

Ar represents naphthyl,

$R^2$ represents fluorine, chlorine, bromine or represents the groupings —X—$R^3$ and —$NR^4R^5$, wherein

$R^3$ represents straight-chain or branched alkyl having 1 to 18 carbon atoms, cycyloalkyl having 3 to 8 carbon atoms, straight-chain or branched alkenyl having 2 to 18 carbon atoms, straight-chain or branched alkinyl having 2 to 18 carbon atoms, hydroxyalkyl having 1 to 18 carbon atoms, alkylthioalkyl having 1 to 6 carbon atoms in the alkylthio group and 1 to 6 carbon atoms in the alkyl group, carboxyalkyl having 1 to 18 carbon atoms in the alkyl part, alkoxycarbonylalkyl having 1 to 6 carbon atoms in the alkoxy part and 1 to 6 carbon atoms in the alkyl part, and phenyl or phenylalkyl having 1 to 2 carbon atoms in the alkyl part, in which each of the phenyl radicals can be monosubstituted or polysubstituted by identical or different substituents, substituents being halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl or phenoxy, or

$R^3$ represents the radical of the formula

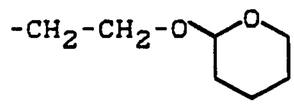

$R^4$ and $R^5$ independently of one another represent hydrogen or straight-chain or branched alkyl having 1 to 4 carbon atoms, or

$R^4$ and $R^5$, together with the nitrogen atom to which they are bonded, represent a 5-membered or 6-membered ring which is optionally substituted by alkyl having 1 to 4 carbon atoms, which can contain oxygen, sulphur and/or nitrogen as further hetero atoms, and

X represents oxygen, sulphur, an SO group or an $SO_2$ group, and

Y represents nitrogen or a CH group,

and their acid addition salts and metal salt complexes, characterized in that

a) in a *first stage*, aryl-cyclopropyl-ketones of the formula

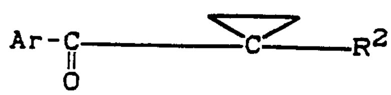

(II)

in which Ar and $R^2$ have the meaning given above,
are reacted with dimethyloxosulphonium methylide of the formula

$$(CH_3)_2{}^{\delta+}SO^{\delta-}CH_2 \qquad (III)$$

in the presence of a diluent, and, in a *second stage*, the resulting aryl-cyclopropyl-oxiranes of the formula

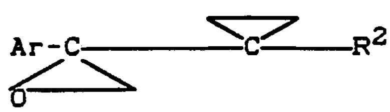

(IV)

in which Ar and $R^2$ have the meaning given above,
are reacted with azoles of the formula

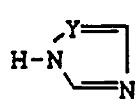

(V)

in which Y has the meaning given above,
in the presence of a diluent and in the presence of a base, or

b) azolylketo compounds of the formula

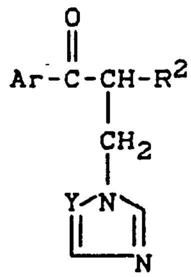

(VI)

in which $R^2$, Ar and Y have the meaning given above, are reacted with dimethyloxosulphonium methylide of the formula

$$(CH_3)_2^{\delta+}SO^{\delta-}CH_2 \qquad (III)$$

in the presence of a diluent, or

c) azolylmethyl-thio-cyclopropyl-carbinol derivatives of the formula

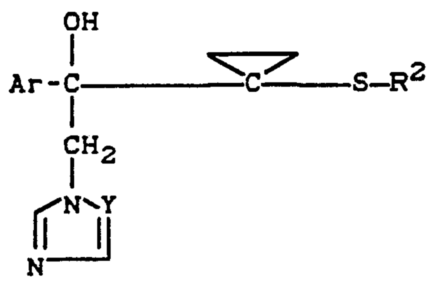

(Ia)

in which Ar, $R^3$, and Y have the meaning given above, are reacted with oxidizing agents, if appropriate in the presence of a diluent, and, if appropriate, the compounds of the formula (I) which are obtained in this manner are then subjected to an addition reaction with an acid or a metal salt.

3. Plant growth-regulating and fungicidal agents, characterized in that they contain at least one substituted azolylmethyl-cyclopropyl-carbinol derivative of the formula (I) according to Claim 1 or one acid addition salt or metal salt complex of a substituted azolylmethyl-cyclopropyl-carbinol derivative of the formula (I).

4. Use of substituted azolylmethyl-cyclopropyl-carbinol derivatives of the formula (I) according to Claim 1 or of their acid addition salts and metal salt complexes for regulating plant growth and for combating phytopathogenic fungi.

5. Aryl cyclopropyl ketones of the formula

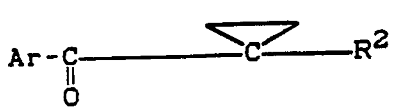

(IIa)

in which

Ar represents phenyl which can be monosubstituted or polysubstituted by identical or different substituents, substituents being: halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl and phenoxy, or

Ar represents naphthyl,

$R^2$ represents fluorine, chlorine, bromine or represents the groupings $—X'—R^3$ and $—NR^4R^5$, wherein

$R^3$ represents straight-chain or branched alkyl having 1 to 18 carbon atoms, cycyloalkyl having 3 to 8 carbon atoms, straight-chain or branched alkenyl having 2 to 18 carbon atoms, straight-chain or branched alkinyl having 2 to 18 carbon atoms, hydroxyalkyl having 1 to 18 carbon atoms, alkylthioalkyl having 1 to 6 carbon atoms in the alkylthio group and 1 to 6 carbon atoms in the alkyl group, carboxyalkyl having 1 to 18 carbon atoms in the alkyl part, alkoxycarbonylalkyl having 1 to 6 carbon atoms in the alkoxy part and 1 to 6 carbon atoms in the alkyl part, and phenyl or phenylalkyl having 1 or 2 carbon atoms in the alkyl part, in

which each of the phenyl radicals can be monosubstituted or polysubstituted by identical or different substituents, substituents being halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl or phenoxy, or

$R^3$ represents the radical of the formula

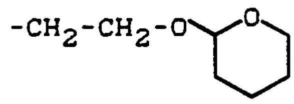

$R^4$ and $R^5$ independently of one another represent hydrogen or straight-chain or branched alkyl having 1 to 4 carbon atoms, or

$R^4$ and $R^5$, together with the nitrogen atom to which they are bonded, represent a 5-membered or 6-membered ring which is optionally substituted by alkyl having 1 to 4 carbon atoms, which can contain oxygen, sulphur and/or nitrogen as further hetero atoms, and

X' represents oxygen, sulphur, an SO group or an $SO_2$ group.

6. Process for the preparation of aryl cyclopropyl ketones of the formula

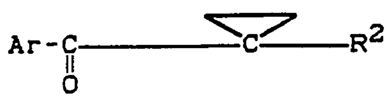

(IIa)

in which

Ar represents phenyl which can be monosubstituted or polysubstituted by identical or different substituents, substituents being: halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl and phenoxy, or

Ar represents naphthyl,

$R^2$ represents fluorine, chlorine, bromine or represents the groupings —X'—$R^3$ and —$NR^4R^5$, wherein

$R^3$ represents straight-chain or branched alkyl having 1 to 18 carbon atoms, cycyloalkyl having 3 to 8 carbon atoms, straight-chain or branched alkenyl having 2 to 18 carbon atoms, straight-chain or branched alkinyl having 2 to 18 carbon atoms, hydroxyalkyl having 1 to 18 carbon atoms, alkylthioalkyl having 1 to 6 carbon atoms in the alkylthio group and 1 to 6 carbon atoms in the alkyl group, carboxyalkyl having 1 to 18 carbon atoms in the alkyl part, alkoxycarbonylalkyl having 1 to 6 carbon atoms in the alkoxy part and 1 to 6 carbon atoms in the alkyl part, and phenyl or phenylalkyl having 1 or 2 carbon atoms in the alkyl part, in which each of the phenyl radicals can be monosubstituted or polysubstituted by identical or different substituents, substituents being halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl or phenoxy, or

$R^3$ represents the radical of the formula

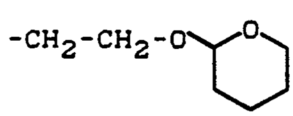

$R^4$ and $R^5$ independently of one another represent hydrogen or straight-chain or branched alkyl having 1 to 4 carbon atoms, or

$R^4$ and $R^5$, together with the nitrogen atom to which they are bonded, represent a 5-membered or 6-membered ring which is optionally substituted by alkyl having 1 to 4 carbon atoms, which can contain oxygen, sulphur and/or nitrogen as further hetero atoms, and

X' represents oxygen, an SO group or an $SO_2$ group, characterized in that aryl halogenopropyl ketones of the formula

Ar—CO—CH—$CH_2CH_2$—Hal''
|
Hal' (VIII)

in which
Ar has the meaning given above,
Hal′ represents fluorine, chlorine or bromine and
Hal″ represents bromine or chlorine, are reacted in the presence of a diluent and in the presence of a base
α) with compounds of the formula

$$HR^6 \qquad (IX)$$

in which
$R^6$ represents the groupings —$X'R^3$ and —$NR^4R^5$, wherein $R^3$, $R^4$, $R^5$ and X′ have the meanings given above,
β) are heated directly in the presence of a diluent and in the presence of a base.

7. Aryl-cyclopropyl-oxiranes of the formula

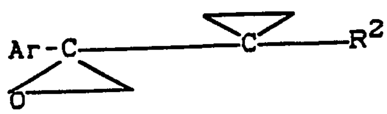

(IV)

in which
Ar represents phenyl which can be monosubstituted or polysubstituted by identical or different substituents, substituents being: halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl and phenoxy, or
Ar represents naphthyl,
$R^2$ represents fluorine, chlorine, bromine or represents the groupings —X—$R^3$ and —$NR^4R^5$, wherein
$R^3$ represents straight-chain or branched alkyl having 1 to 18 carbon atoms, cycyloalkyl having 3 to 8 carbon atoms, straight-chain or branched alkenyl having 2 to 18 carbon atoms, straight-chain or branched alkinyl having 2 to 18 carbon atoms, hydroxyalkyl having 1 to 18 carbon atoms, alkylthioalkyl having 1 to 6 carbon atoms in the alkylthio group and 1 to 6 carbon atoms in the alkyl group, carboxyalkyl having 1 to 18 carbon atoms in the alkyl part, alkoxycarbonylalkyl having 1 to 6 carbon atoms in the alkoxy part and 1 to 6 carbon atoms in the alkyl part, and phenyl or phenylalkyl having 1 or 2 carbon atoms in the alkyl part, in which each of the phenyl radicals can be monosubstituted or polysubstituted by identical or different substituents, substituents being halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl or phenoxy, or
$R^3$ represents the radical of the formula

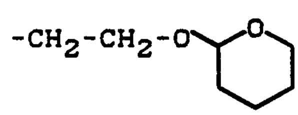

$R^4$ and $R^5$ independently of one another represent hydrogen or straight-chain or branched alkyl having 1 to 4 carbon atoms, or
$R^4$ and $R^5$, together with the nitrogen atom to which they are bonded, represent a 5-membered or 6-membered ring which is optionally substituted by alkyl having 1 to 4 carbon atoms, which can contain oxygen, sulphur and/or nitrogen as further hetero atoms, and
X represents oxygen, sulphur, an SO group or an $SO_2$ group.

8. Process for the preparation of aryl-cyclopropyl-oxiranes of the formula

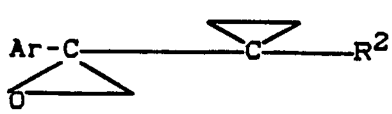

(IV)

in which
Ar represents phenyl which can be monosubstituted or polysubstituted by identical or different substituents, substituents being: halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or

different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl and phenoxy, or

Ar represents naphthyl,

$R^2$ represents fluorine, chlorine, bromine or represents the groupings —X—$R^3$ and —$NR^4R^5$, wherein

$R^3$ represents straight-chain or branched alkyl having 1 to 18 carbon atoms, cycyloalkyl having 3 to 8 carbon atoms, straight-chain or branched alkenyl having 2 to 18 carbon atoms, straight-chain or branched alkinyl having 2 to 18 carbon atoms, hydroxyalkyl having 1 to 18 carbon atoms, alkylthioalkyl having 1 to 6 carbon atoms in the alkylthio group and 1 to 6 carbon atoms in the alkyl group, carboxyalkyl having 1 to 18 carbon atoms in the alkyl part, alkoxycarbonylalkyl having 1 to 6 carbon atoms in the alkoxy part and 1 to 6 carbon atoms in the alkyl part, and phenyl or phenylalkyl having 1 or 2 carbon atoms in the alkyl part, in which each of the phenyl radicals can be monosubstituted or polysubstituted by identical or different substituents, substituents being halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, phenyl or phenoxy, or

$R^3$ represents the radical of the formula

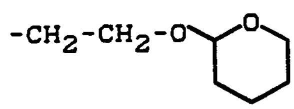

$R^4$ and $R^5$ independently of one another represent hydrogen or straight-chain or branched alkyl having 1 to 4 carbon atoms, or

$R^4$ and $R^5$, together with the nitrogen atom to which they are bonded, represent a 5-membered or 6-membered ring which is optionally substituted by alkyl having 1 to 4 carbon atoms, which can contain oxygen, sulphur and/or nitrogen as further hetero atoms, and

X represents oxygen, sulphur, an SO group or an $SO_2$ group,

characterized in that aryl cyclopropyl ketones of the formula

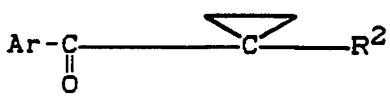

(II)

in which

Ar and $R^2$ have the meaning given above,

are reacted with dimethyloxosulphonium methylide of the formula

$$(CH_3)_2^{\delta+}SO^{\delta-}CH_2 \qquad (III)$$

in the presence of a diluent.